# EUROPEAN PATENT SPECIFICATION

(11) **EP 0 713 533 B1**
(45) Date of publication and mention of the grant of the patent: **10.07.2002**
(21) Application number: 94925870.1
(22) Date of filing: 12.08.1994
(51) Int. Cl.: C12P 1/00, C12Q 1/00, G01N 33/50, C12P 19/32, C12P 17/02

(54) **BIOCATALYTIC METHODS FOR SYNTHESIZING AND IDENTIFYING BIOLOGICALLY ACTIVE COMPOUNDS**
BIOKATALYTISCHE METODEN ZUM SYNTHETISIEREN UND IDENTIFIZIEREN BIOLOGISCH AKTIVE VERBINDUNGEN
PROCEDES BIOCATALYTIQUES DE SYNTHESE ET D'IDENTIFICATION DE COMPOSES BIOLOGIQUEMENT ACTIFS

(30) Priority: 13.08.1993 US 106279
(43) Date of publication of application: 29.05.1996
(73) Proprietor: ALBANY MOLECULAR RESEARCH, INC., Albany, NY 12203 (US)
(72) Inventor: FOX, J. Wesley, Schaumburg, IL 60194 (US); DORDICK, Jonathan S., Iowa City, IA 52240 (US); CLARK, Douglas S., Oakland, CA 94611 (US)
(74) Representative: Orès, Bernard
(86) International application number: US9409174
(87) International publication number: WO9505475

(56) References cited:
- WO-A-92/00091
- PROC. NATL. ACAD. SCI., Volume 89, 1992, (05), Ronald N. Zuckermann et al, "Identification of highest-affinity ligands by affinity selection from equimolar peptide mixtures generated by robotic synthesis"
- LETTERS TO NATURE, Volume 354, November 1991, Kit S. Lam et al, "A new type of synthetic peptide library for identifying ligand-binding activity"
- PROC. NATL. ACAD. SCI., Volume 90, August 1993, Sheila Hobbs De Witt et al, "Diversomers": an approach to nonpeptide, nonoligomeric chemical diversity"

## Description

### BACKGROUND OF THE INVENTION

### (a) Field of the Invention

This invention is in the field of synthesizing and identifying biologically active compounds.

### (b) Description of the Prior Art

The prior art is repleat with examples of chemically, microbially, or enzymatically synthesizing compounds with biological activity. The goal of these efforts is the discovery of new and improved pharmaceutical compounds.

The discovery of new pharmaceutical compounds is for the most part a trial and error process. So many diverse factors constitute an effective pharmaceutical compound that it is extremely difficult to reduce the discovery process to a systematic approach. Typically, thousands of organic compounds must be isolated from biological sources or chemically synthesized and tested before a pharmaceutical compound is found.

Synthesizing and testing new compounds for biological activity, which is the first step in identifying a new synthetic drug, is a time consuming and expensive undertaking. Typically, compounds must by synthesized, purified, tested and quantitatively compared to other compounds in order to identify active compounds or identify compounds with optimal activity. The synthesis of new compounds is accomplished for the most part using standard chemical methods. Such methods provide for the synthesis of virtually any type of organic compound; however, because chemical reactions are non-specific, these syntheses require numerous steps and multiple purifications before a final compound is produced and ready for testing.

New biological and chemical approaches have recently been developed which provide for the synthesis and screening of large libraries of small peptides and oligonucleotides. These methods provide for the synthesis of a broad range of chemical compounds and provide the means to potentially identify biologically active compounds. The chemistries for synthesizing such large numbers of these natural and non-naturally occurring polymeric compounds is complicated, but manageable because each compound is synthesized with the same set of chemical protocols, the difference being the random order in which amino acids or nucleotides are introduced into the reaction sequence.

Fodor, S.P.A. et al (1990) Science 251, 767-773, describe methods for discovering new peptide ligands that bind to biological receptors. The process combines solid-phase chemistry and photolithography to achieve a diverse array of small peptides. This work and related works are also described in Fodor WO Patent #9,210,092, Dower WO #9,119,818. Barrett WO #9,107,087 and Pirrung WO#9,015,070.

Houghten, R.A. et al. (1991) Nature 354, 84-86, describe an approach that synthesizes libraries of free peptides along with an iterative selection process that permits the systematic identification of optimal peptide ligands. This work is also described in Appl. WO Patent #9,209,300.

Lam, K.S., et al. (1991) Nature 354, 82-84, describe a method that provides for the systematic synthesis and screening of peptide libraries on a solid-phase microparticle support on the basis of a 'one-bead, one-peptide' approach.

Cwirla, S.E., et al. (1990) Proc. Natl. Acad. Sci. USA 87, 6378-6382, describe a method for constructing a library of peptides on the surface of a phage by cloning randomly synthesized oligonucleotides into the 5' region of specific phage genes resulting in millions of different hexapeptides expressed at the N terminus of surface proteins.

The International Application WO 91/12331 discloses a method for generating and screening useful peptides, said method comprising a scrambling reaction involving one or more non-specific proteases so as to generate a multiplicity of peptides containing greatly varied combinations of amino acids, the removal of the so-generated peptides from the scrambling system by a specific binding to a macromolecule trap that is separated from the scrambling system by a semi-permeable barrier which allows passage of peptides up to a particular size without allowing passage of macromolecules, including the proteases, the separation of the peptides from the complexes resulting from this specific binding and then the identification of said peptides.

These methods accelerate the identification of biologically active peptides and oligonucleotides. However, peptides and oligonucleotides have poor bioavailability and limited stability in vivo, which limits their use as therapeutic agents. In general, non-biological compounds which mimic the structure of the active peptides and oligonucleotides must be synthesized based on the approximated three dimensional structure of the peptide or oligonucleotide and tested before an effective drug structure can be identified.

Bunin et al., J. Am. Chem. Soc. (1992) 114, 10997-10998 describe the synthesis of numerous 1, 4 benzodiazapine derivatives using solid phase synthesis techniques.

The prior art is repleat with examples showing enzymatic conversion of non-physiological substances under many conditions.

### References demontrating that enzyme specificity can be changed/tailored :

1. Zaks, A. and Klibanov, A.M. Substrate specificity of enzymes in organic solvents vs. water is reversed. Journal of the American Chemical Society 108 2767-2768, 1986.
2. Ferjancic, A., Puigserver, A. and Gaertner, H. Unusual specificity of PEG-modified thermolysin in peptide synthesis catalyzed in organic solvents. Biotechnology Letters 10 (2) 101-106, 1988.
3. Narsi, M. and Thomas, D. Increase of the potentialities of restriction endonuleases by specificity relaxation in the presence of organic solvents. Ann. N.Y. Acad. Sci. 542 255-265, 1988.
4. Stahl, M., Mansson, M.O. and Mosbach, K. The synthesis of a D-amino acid ester in an organic media with chymotrypsin modified by a bioimpriting procedure. Biotechnology Letters 12 (3) 161-166, 1990.
5. Stahl, M., Jeppsson-Wistrand, U., Mansson, M.O. and Mosbach, K. Induced stereoselectivity and substrate selectivity of bio-imprinted a-chymotrypsin in anhydrous organic media. Journal of the American Chemical Society 113 (24) 9366-9368, 1991.
6. Gololobov, M.Y., Voyushina, T.L., Stepanov, V.M. and Adlercreutz, P. Organic solvent changes the chymotrypsin specificity with respect to nucleophiles, FEBS Letters 307 (3) 309-312, 1992.
7. Hertmanni, P., Pourplanche, C. and Larreta-Garde, V. Orientation of enzyme catalysis and specificity by water-soluble additives. Ann. New York Acad.Sci. (Enzyme Eng. XI, D.S. Clark, D.A. Estell, eds) 672 329-335, 1992.
8. Cabezas, M.J., del Campo, C., Llama, E., Sinisterra, J.V. and Gaertner, H. Organic reactions catalysed by modified enzymes. 1. Alteration of the substrate specificity of a-chymotrypsin by the modification process. Journal of Molecular Catalysis 71 (2) 261-278, 1992.
9. Nagashima, T., Watanabe, A. and Kise, H. Peptide synthesis by proteases in organic solvents: medium effect on substrate specificity. Enzyme and Microbial Technology 14 (10) 842-847, 1992.
10. Parida, S. and Dordick, J.S. Tailoring lipase specificity by solvent and substrate chemistries, J. Org. Chem. 58 (12) 3238-3244, 1993.
11. Tawaki, S. and Klibanov, A.M. Chemoselectivity of enzymes in anhydrous media is strongly solvent dependent. Biocatalysis 8 (1) 3-19, 1993.
12. Wescott, C.F. and Klibanov, A.M. Solvent variation inverts substate specificity of an enzyme. JACS 115 (5) 1629-1631, 1993.

### References demonstrating that enzyme enantioselectivity can be changed/tailored:

1. Sakurai, T., Margolin, A.L., Russell, A.J. and Klibanov, A. M. Control of enzyme enantioselectivity by the reaction medium. Journal of the American Chemical Society 110 (21) 7236-7237, 1988.
2. Fitzpatrick, P.A. and Klibanov, A.M. How can the solvent affect enzyme enantioselectivity? Journal of the American Chemical Society 113 (8) 3166-3171, 1991.
3. Hult, K. and Norin, T. Enantioselectivity of some lipases - control and prediction. Pure and Applied Chemistry 64 (8) 1129-1134, 1992.
4. Miyazawa, T., Kurita, S., Ueji, S., Yamada, T. and Kuwata, S. Resolution of racemic carboxylic acids via the lipase-catalyzed irreversible transesterification using vinyl esters-effects of alcohols as nucleophiles and organic solvents on enantioselectivity. Biotechnology Letters 14 (10) 941-946, 1992.
5. Tawaki, S. and Klibanov, A.M. Inversion of enzyme enantioselectivity mediated by the solvent. Journal of the American Chemical Society 114(5) 1882-1884, 1992.
6. Ueji, S., Fujino, R., Okubo, N., Miyazawa, T., Kurita, S., Kitadani, M. and Muromatsu, A. Solvent-induced inversion of enantioselectivity in lipase-catalyzed esterification of 2-phenoxypropionic acids. Biotechnology Letters 14 (3) 163-168, 1992.
7. Terradas, F., Testonhenry, M., Fitzpatrick, P.A. and Klibanov, A.M. Marked dependence of enzyme prochiral selectivity on the solvent. Journal of the American Chemical Society 115 (2) 390-396, 1993.
8. Herradon, B. Biocatalytic synthesis of chiral polyoxygenated compounds: effect of the solvent on the enantioselectivity of lipase catalyzed transesterifications in organic solvents. Synlett 2 108-110, 1993.

### References demonstrating the ability of enzymes to convert unnatural substrates:

1. Bianchi, D., Cesti, P., Golini, P., Spezia, S., Garavaglia, C. and Mirenna, L. Enzymatic preparation of optically active fungicide intermediates in aqueous and in organic media. Pure and Applied Chemistry 64 (8) 1073-1078, 1992.
2. Natoli, M., Nicolosi, G. and Piattelli, M. Regioselective alcoholysis of flavonoid acetates with lipase in an organic solvent. Journal of Organic Chemistry 57 (21) 5776-5778, 1992.
3. Izumi, T., Tamura, F. and Sasaki, K. Enzymatic kinetic resolution of <4>(1,2)ferrocenophane derivatives. Bulletin of the Chemical Society of Japan 65 (10) 2784-2788, 1992.
4. Miyazawa, T., Mio, M., Watanabe, Y., Yamada, T. and Kuwata, S. Lipase-catalyzed transesterification procedure for the resolution of non-protein amino acids. Biotechnology Letters 14 (9) 789-794, 1992.
5. Murata, M., Uchida, H. and Achiwa, K. Lipase-catalyzed enantioselective synthesis of optically active mephobarbital, hexobarbital and febarbamate. Chemical-Pharmaceutical Bulletin 40 (10) 2605-2609, 1992.
6. Johnson, C.R., Golebiowski, A. and Steensma, D.H. Enzymatic asymmetrization in organic media - synthesis of unnatural glucose from cycloheptatriene. Journal of the American Chemical Society 114 (24) 9414-9418, 1992.
7. Cruces, M.A., Otero, C., Bernabe, M., Martinlomas, M. and Ballesteros, A. Enzymatic preparation of acylated sucroses. Ann. New York Acad.Sci. (Enzyme Eng. XI, D.S. Clark, D.A. Estell, eds) 672 436-443, 1992.
8. Tanaka, A., Fukui, T., Uejima, A., Zong, M.H. and Kawamoto, T. Bioconversion of nonnatural organic compounds - esterification and dehydrogenation of organosilicon compounds. Ann. New York Acad. Sci (Enzyme Eng. XI, D.S. Clark, D.A. Estell, eds) 672 431-435, 1992.
9. Kodelia, G. and Kolisis, F.N. Studies on the reaction catalyzed by protease for the acylation of flavonoids in organic solvents. Ann. New York Acad.Sci (Enzyme Eng. XI, D.S. Clark, D.A. Estell, eds) 672 451-457, 1992.
10. Wagner, F., Kleppe, F., Lokotsch, W., Ziemann, A. and Lang, S. Synthesis of uncommon wax esters with immobilized lipases. Ann. New York Acad.Sci. (Enzyme Eng. XI, D.S. Clark, D.A. Estell, eds) 672 484-491, 1992.
11. Patel. R.N., Howell, J.M., Banerjee, A., Fortney, K.F. and Szarka, L. J. Stereoselective enzymatic esterification of 3-benzoylthio-2-methylpropanoic acid. Ann. New York Acad. Sci (Enzyme Eng. XI, D.S. Clark, D.A. Estell, eds) 672 415-424, 1992.
12. Bergbreiter, D.E. and Momongan, M. Asymmetric synthesis of organometallic reagents using enzymatic methods. Applied Biochemistry and Biotechnology 32 1-3 55-72, 1992.
13. Carretero, J.C. and Dominguez, E. Lipase-catalyzed kinetic resolution of hydroxy phenyl sulfones. Journal of Organic Chemistry 57 (14) 3867-3873, 1992.
14. Johnson, C.R., Adams, J.P., Bis, S.J., Dejong, R.L., Golebiowski, A., Medich, J. R., Penning, T.D., Senanayake, C.H., Steensma, D.H. and Vanzandt, M.C. Applications of enzymes in the synthesis of bioactive polyols. Indian Journal of Chemistry Section B - Organic Chemistry Including Medicinal Chemistry 32 (1) 140-144, 1993.
15. Fabre, J., Betbeder, D., Paul, F., Monsan, P. and Perie, J. Regiospecific enzymic acylation of butyl a-D-glucopyranoside. Carbohydrate Research 243 (2) 407-411, 1993.
16. Bianchi, D., Cesti, P., Golini, P., Spezia, S., Garavaglia, C. and Mirenna, L. Enzymatic preparation of optically active fungicide intermediates in aqueous and in organic media. Indian Journal of Chemistry Section B - Organic Chemistry Including Medicinal Chemistry 32 (1) 176-180, 1993.
17. Kanerva, L.T. and Sundholm, O. Lipase catalysis in the resolution of racemic intermediates of diltiazem synthesis in organic solvents. Journal of the Chemical Society - Perkin Transactions I 13 1385-1389, 1993.
18. Ikeda, I. and Klibanov, A.M. Lipase-catalyzed acylation of sugars solubilized in hydrophobic solvents by complexation. Biotechnology and Bioengineering 42(6) 788-791, 1993.
19. Hyun, C.K., Kim, J.H. and Ryu, D.D.Y. Enhancement effect of water activity on enzymatic synthesis of cephalexin. Biotechnology and Bioengineering 42(7) 800-806, 1993.
20. Panza, L., Luisetti, M., Crociati, E. and Riva, S. Selective acylation of 4,6-O-benzylidene glycopyranosides by enzymatic catalysis. J. Carbohydrate Chem. 12 (1) 125-130, 1993.
21. Wang, L., Kobatake, E., Ikariyama, Y. and Aizawa, M. Regioselective oxidative polymerization of 1,5- dihydroxynaphthalene catalyzed by bilirubin oxidase in a water-organic solvent mixed solution. Journal of Polymer Science Part A - Polymer Chemistry 31 (11) 2855-2861, 1993.
22. Knani, D. and Kohn, D.H. Enzymatic polyesterification in organic media. 2. Enzyme-catalyzed synthesis of lateral-substituted aliphatic polyesters and copolyesters. J. Polymer Sci., Part A - Polymer Chem. 31 (12) 2887-2897, 1993.
23. Kanerva, L.T. and Sundholm O. Enzymatic acylation in the resolution of methyl threo-2-hydroxy-3-(4-methoxyphenyl)-3-(2-x-phenylthio) propionates in organic solvents. Journal of the Chemical Society - Perkin Transactions I 20 2407-2410, 1993.
24. Sharma, A. and Chattopadhyay, S. Lipase catalysed acetylation of carbohydrates. Biotechnology Letters 15 (11) 1145-1146, 1993.
25. Pavel, K. and Ritter, H. Enzymes in polymer chemistry. 7. Lipase-catalyzed esterification of carboxyl-terminated methacrylic oligomers and copolymers with isopropyl alcohol and 9-fluorenylmethanol. Makromolekulare Chemie- Macromolecular Chemistry and Physics 194 (12) 3369-3376, 1993.
26. Uejima, A., Fukui, T., Fukusaki, E., Omata, T., Kawamoto, T., Sonomoto, K. and Tanaka, A. Efficient kinetic resolution of organosilicon compounds by stereoselective esterification with hydrolases in organic solvent. Appl. Microbiol.Biotech. 38 (4) 482-486, 1993.
27. Mukesh, D., Sheth, D., Mokashi, A., Wagh, J., Tilak, J.M., Banerji, A.A. and Thakkar, K.R. Lipase catalysed esterification of isosorbide and sorbitol. Biotechnology Letters 15 (12) 1243-1246, 1993.
28. Baldessari, A., Iglesias, L.E. and Gros, E.G. Regioselective acylation of 3-mercaptopropane-1,2-diol by lipase-catalysed transesterification. Journal of Chemical Research-S 9 382-383, 1993.
29. De Goede, A.T.J.W., Benckhuijsen, W., van Rantwijk, F., Maat, L. and van Bekkum, H. Selective lipase-catalyzed 6-O-acylation of alkyl a-D-glucopyranosides using functionalized ethyl esters. Recueil Des Travaux Chimiques Des Pays Bas - Journal of the Royal Netherlands Chemical Society 112 (11) 567-572, 1993.
30. Menendez,E. and Gotor, V. Acylation and alkoxycarbonylation of oximes through an enzymatic oximolysis reaction. Synthesis - Stuttgart 1 72-74, 1993.
31. Li, Y.F. and Hammerschmidt, F. Enzymes in organic chemistry. 1. Enantioselective hydrolysis of a-(acyloxy) phosphonates by esterolytic enzymes. Tetrahedron-Asymmetry 4 (1) 109-120, 1993.
32. Schlotterbeck, A., Lang, S., Wray, V. and Wagner, F. Lipase-catalyzed monoacylation of fructose. Biotechnology Letters 15 (1) 61-64, 1993.
33. Frykman, H., Ohrner, N., Norin, T. and Hult, K. S-Ethyl thiooctanoate as acyl donor in lipase catalysed resolution of secondary alcohols. Tetrahedron Letters 34 (8) 1367-1370, 1993.
34. Oguntimein, G.B., Erdmann, H. and Schmid, R.D. Lipase catalysed synthesis of sugar ester in organic solvents. Biotechnology Letters 15 (2) 175-180, 1993.
35. Lopez, R., Perez, C., Fernandez-Mayorales, A. and Conde, S. Enzymatic transesterification of alkyl 2,3,4-tri-O-acyl-beta-D-xylopyranosides. J. Carbohydrate Chem. 12 (2) 165-171, 1993.
36. Naemura, K., Ida. H. and Fukuda, R. Lipase YS-catalyzed enantioselective transesterification of alcohols of bicarbocyclic compounds. Bull. Chem. Soc.Japan 66 (2) 573-577, 1993.
37. Lambusta, D., Nicolosi, G., Piattelli, M. and Sanfilippo, C. Lipase catalyzed acylation of phenols in organic solvents. Ind. J .Chem. Section B 32 (1) 58-60, 1993.
38. Astorga, C., Rebolledo, F. and Gotor, V. Enzymatic hydrazinolysis of diesters and synthesis of N-aminosuccinimide derivatives. Synthesis - Stuttgart 3 287-289, 1993.
39. Fukui, T., Kawamoto, T. and Tanaka. A. Enzymatic preparation of optically active silylmethanol derivatives having a stereogenic silicon atom by hydrolase-catalyzed enantioselective esterification. Tetrahedron - Asymmetry 5 (1) 73-82, 1994.
40. Vazquez-Duhalt, R., Westlake, D.W.S. and Fedorak, P.M. Lignin peroxidase oxidation of aromatic compounds in systems containing organic solvents. Applied and Environmental Microbiology 60 (2) 459-466, 1994.
41. Kodelia, G., Athanasiou, K. and Kolisis, F.N. Enzymatic synthesis of butyrylrutin ester in organic solvents and its cytogenetic effects in mammalian cells in culture. Appl. Biochem. Biotech. 44 (3) 205-212, 1994.
42. Tsai, S.W. and Wei, H.J. Effect of solvent on enantioselective esterification of naproxen by lipase with trimethylsilyl methanol. Biotechnology and Bioengineering 43 (1) 64-68, 1994.
43. Athawale, V.D. and Gaonkar, S.R. Enzymatic synthesis of polyesters by lipase catalysed polytransesterification. Biotechnology Letters 16 (2) 149-154, 1994.
44. Janssen, G.G. and Haas, MJ. Lipase-catalyzed synthesis of oleic acid esters of polyethylene glycol 400. Biotechnology Letters 16 (2) 163-168, 1994.
45. Kitazuma, T., Ikeya, T. and Murata, K. Synthesis of optically active trifluorinated compounds: asymmetric Michael additional with hydrolytic enzymes. Journal of Chemical Society, Chemical Communications 1331, 1986.

### References demonstrating microbial transformations:

1. Holland, H.L. Fungi as reagents in organic synthesis: preparation of some metabolites of prostanoids, steroids, and other natural products. Rev. Latinoam. Quim., (1st Spec. Suppl.), 318-329, 1990.
2. Sih, C.J. and Rosazza, J.P. Microbial transformations in organic synthesis. Tech. Chem. (N.Y.), 10 (Appl. Biochem. Syst. Org. Chem., Part 1) 69-106, 1976.
3. Sih, C.J. and Chen, C.S. Microbial asymmetric catalysis-enantioselective reduction of ketones. Angew. Chem. Int. Ed. Engl. 23 (8) 570-578, 1984.
4. Thompson, L.A. and Knowles, C.J., Linton, E.A. and Wyatt, J.M. Microbial biotransformations of nitriles. Chem. Br. 24 (9) 900, 1988.
5. Servi, S. Baker's yeast as a reagent in organic synthesis. Synthesis 1 1-25, 1990.
6. Csuk, R. and Glanzer, B.I. Baker's yeast mediated transformations in organic chemistry. Chem. Rev. 91 (1) 49-97, 1991.
7. Roberts, S.M., Wiggins, K. and Casy, G. *Whole cells and isolated enzymes in organic synthesis*. Wiley, New York, 1992.
8. Ward, O.P. and Young, C.S. Reductive biotransformations of organic compounds by cells or enzymes of yeast. Enxyme Microb. Technol. 12 (7) 482-493, 1990.

### Reviews:

1. Jones, J.B. Enzymes in organic synthesis. Tetrahedron 42 (13) 3351-3405, 1986.
2. Yamada, H. and Shimizu, S. Microbial and enzymatic processes for the production of biologically and chemically useful compounds. Angew. Chem. Int. Ed. Engl. 27 (5) 622-642, 1988.
3. Roberts, S.M. Enzymes as catalysts in organic synthesis. NATO ASI Ser., Ser. A. 178 443-463, 1989.
4. Chen, S.S. and Sih, C.J. General aspects and optimization of enantioselective biocatalysis in organic solvents: the use of lipases. Angew. Chem (Int. Ed. Engl. 28, n 6 695-707) 101 (6) 711-724, 1989.

### SUMMARY OF THE INVENTION

The present invention is used to synthesize a library of non-biological organic compounds from a starting compound and identify individual compounds within the library which exhibit biological activity. Unlike peptides and oligonucleotides, non-biological organic compounds comprise the bulk of proven therapeutic agents. The invention can be used to directly identify new drug candidates or optimize an established drug compound which has sub-optimal activity or problematic side effects. This is accomplished through the use of highly specific biocatalytic reactions.

Enzymes are highly selective catalysts. Their hallmark is the ability to catalyze reactions with exquisite stereo-, regio-, and chemo-selectivities that are unparalleled in conventional synthetic chemistry. Moreover, enzymes are remarkably versatile. They can be tailored to function in organic solvents, operate at extreme pH's and temperatures, and catalyze reactions with compounds that are structurally unrelated to their natural, physiological substrates.

Enzymes are reactive toward a wide range of natural and unnatural substrates, thus enabling the modification of virtually any organic lead compound. Moreover, unlike traditional chemical catalysts, enzymes are highly enantio- and regio-selective. The high degree of functional group specificity exhibited by enzymes enables one to keep track of each reaction in a synthetic sequence leading to a new active compound. Enzymes are also capable of catalyzing many diverse reactions unrelated to their physiological function in nature. For example, peroxidases catalyze the oxidation of phenols by hydrogen peroxide. Peroxidases can also catalyze hydroxylation reactions that are not related to the native function of the enzyme. Other examples are proteases which catalyze the breakdown of polypeptides. In organic solution some proteases can also acylate sugars, a function unrelated to the native function of these enzymes.

The present invention exploits the unique catalytic properties of enzymes. Whereas the use of biocatalysts (i.e., purified or crude enzymes, non-living or living cells) in chemical transformations normally requires the identification of a particular biocatalyst that reacts with a specific starting compound, the present invention uses selected biocatalysts and reaction conditions that are specific for functional groups that are present in many starting compounds. Each biocatalyst is specific for one functional group, or several related functional groups, and can react with many starting compounds containing this functional group.

The biocatalytic reactions produce a population of derivatives from a single starting compound. These derivatives can be subjected to another round of biocatalytic reactions to produce a second population of derivative compounds. Thousands of variations of the original compound can be produced with each iteration of biocatalytic derivatization.

Enzymes react at specific sites of a starting compound without affecting the rest of the molecule, a process which is very difficult to achieve using traditional chemical methods. This high degree of biocatalytic specificity provides the means to identify a single active compound within the library. The library is characterized by the series of biocatalytic reactions used to produce it, a so called "biosynthetic history". Screening the library for biological activities and tracing the biosynthetic history identifies the specific reaction sequence producing the active compound. The reaction sequence is repeated and the structure of the synthesized compound determined. This mode of identification, unlike other synthesis and screening approaches, does not require immobilization technologies, and compounds can be synthesized and tested free in solution using virtually any type of screening assay. It is important to note, that the high degree of specificity of enzyme reactions on functional groups allows for the "tracking" of specific enzymatic reactions that make up the biocatalytically produced library.

Many of the procedural steps are performed using robotic automation enabling the execution of many thousands of biocatalytic reactions and screening assays per day as well as ensuring a high level of accuracy and reproducibility. As a result, a library of derivative compounds can be produced in a matter of weeks which would take years to produce using current chemical methods.

The present invention is unique in that it involves a soluble state of the starting compound and its subsequent derivatives. This is a highly unique aspect of the present invention that has been thought to be a barrier. Previous organic modifying technologies for biologically active compound identification involve starting compounds and derivatives attached to insoluble supports. This is taught in examples by Ellman (Bunin, B.S.; Ellman, J.A. "A general and expedient method for the solid-phase synthesis of 1,4-benzodiazepine derivatives", J. Am. Chem. Soc. 1992, 114 10997-10998), Gordon et al., (Gordon, E.M.; Barrett, R.W.; Dower, W.J.; Fodor, S.P.A.; Gallop, M.A. "Applications of Combinatorial Technologies to Drug Discovery, 1. Background and Peptide Combinatorial Libraries", J. Med. Chem. 1994, 37 1233-1251; and Gordon, E.M.; Barrett, R.W.; Dower, W.J.; Fodor, S.P.A.; Gallop, M.A. "Applications of Combinatorial Technologies to Drug Discovery, 2. "Combinatorial Organic Synthesis, Library Screening Strategies, and Future Directions", J. Med. Chem. 1994, 37 1385-1401), Hobbs Dewitt et al., (Hobbs Dewitt, S.; Kiely, J.S.; Stankovic, C.J.; Schroeder, M.C.; Reynolds Cody, D.M.; Pavia, M.R. "Diversomers: An approach to nonpeptide, nonoligomeric chemical diversity" P.N.A.S. (USA) 1993 90 6909-6913). In addition to synthesising the libraries on a soluble state, the libraries of the instant invention can then be screened immediately without removal of the libraries from solid supports. Thus by maintaining the components in the soluble state, the present invention provides for useful, convienent, and efficient methods of generating and screening libraries of starting compounds and derivatives.

The present invention specifically incorporates a number of diverse technologies such as: (1) the use of enzymatic reactions to produce a library of drug candidates; (2) the use of enzymes free in solution or immobilized on the surface of particles, and organic compounds derivatized while dissolved in solution; (3) the use of receptors (hereinafter this term is used to indicate true receptors, enzymes, antibodies and other biomolecules which exhibit affinity toward biological compounds, and other binding molecules to identify a promising drug candidate within a library, even where such receptors are still associated with cell membranes, or intact cells); (4) the automation of all biocatalytic processes and many of the procedural steps used to test the libraries for desired activities, and (5) the coupling of biocatalytic reactions with drug screening devices which can immediately measure the binding of synthesized compounds to receptor molecules or in whole-cell assays and thereby immediately identify specific reaction sequences giving rise to biologically active compounds.

Specifically, the present invention encompasses a method for drug identification comprising:
(a) conducting a series of biocatalytic reactions by mixing biocatalysts with a starting compound to produce a reaction mixture and thereafter a library of modified starting compounds;
(b) testing the library of modified starting compounds to determine if a modified starting compound is present within the library which exhibits a desired activity;
(c) identifying the specific biocatalytic reactions which produce the modified starting compound of desired activity by systematically eliminating each of the biocatalytic reactions used to produce a portion of the library and testing the compounds produced in the portion of the library for the presence or absence of the modified starting compound with the desired activity; and
(d) repeating the specific biocatalytic reactions which produce the modified compound of desired activity and determining the chemical composition of the reaction product.

More specifically, the enzymatic reactions are conducted with a group of enzymes that react with distinct structural moieties found within the structure of a starting compound. Each enzyme is specific for one structural moiety or a group of related structural moieties. Furthermore, each enzyme reacts with many different starting compounds which contain the distinct structural moiety. In addition to systematically eliminating each reaction, the instant invention also provides for the systematic process of building up each reaction, an approach from the other end of the pathway. A particular feature of the instant invention is the soluble state of the starting compounds and the subsequent derivatives.

### BRIEF DESCRIPTION OF THE DRAWINGS

Figure 1 shows the starting active compound AZT with four potential sites for biocatalytic derivatization and eight possible biocatalytic reactions that can be used to produce a library of derivative compounds.
Figure 2 shows an automated system employing robotic automation to perform hundreds of biocatalytic reactions and screening assays per day.
Figure 3 illustrates the tracking of biocatalytic reactions to identify the sequence of reactions producing an active compound, which can subsequently be used to produce and identify the structure of the active compound.
Figure 4a illustrates biocatalytic modification of castanospermine.
Figure 4b illustrates biocatalytic modifications of methotrexate.

### DETAILED DESCRIPTION OF THE INVENTION

While the invention will be described in connection with certain preferred embodiments, it will be understood that the description does not limit the invention to these particular embodiments. The embodiements of the invention are described with AZT, and further described for taxol, only as particular embodiments of the instant invention. In fact, it is to be understood that all alternatives, modifications and equivalents are included and are protected, consistent with the spirit and scope of the inventions as defined in the appended claims.

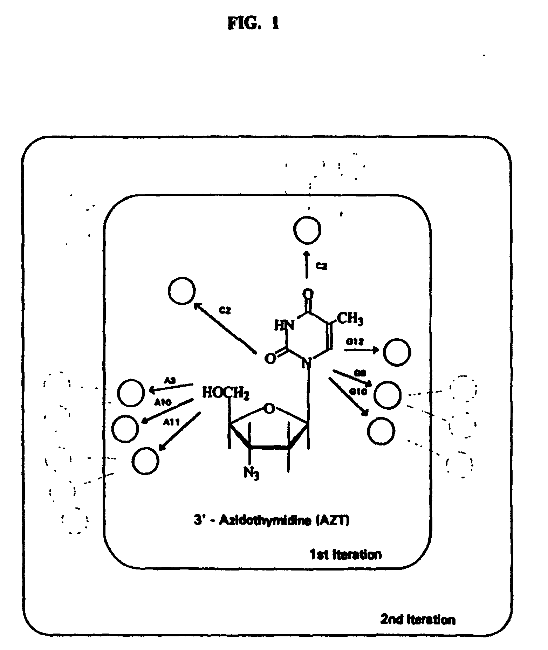

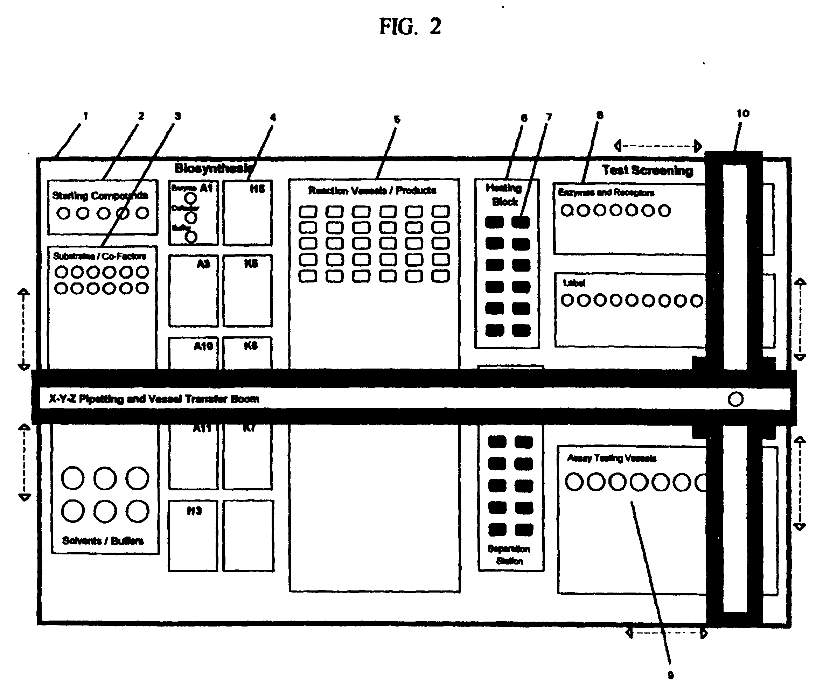

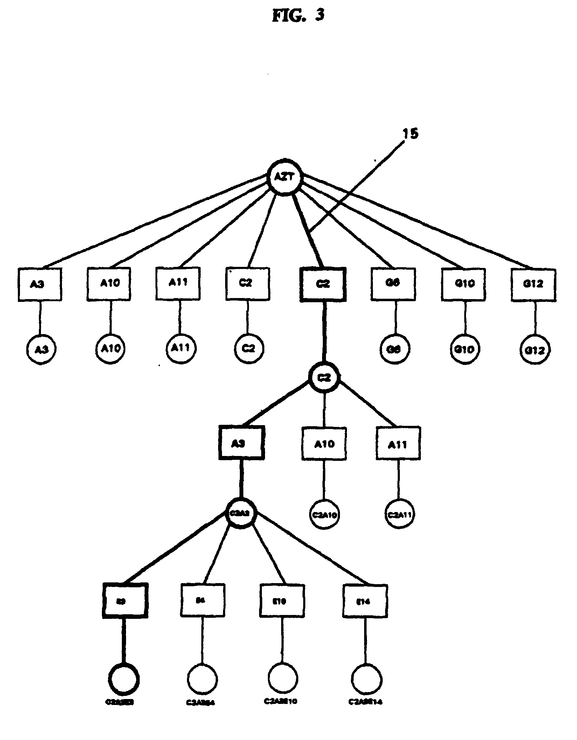

The preferred embodiments of the invention are set forth in the following example:
a) A starting compound such as AZT (3'-azidothymidine), is chosen which exhibits drug activity or is believed to exhibit drug activity for a given disease or disorder. The compound is analyzed with respect to its functional group content and its potential for structural modifications using selected biocatalytic reactions. Functional groups which can be chemically modified using the selected biocatalytic reactions are listed in Table I. One of more of these functional groups are present in virtually all organic compounds. A partial list of possible enzymatic reactions that can be used to modify these functional groups is presented in Table II.
b) A strategy is developed to systematically modify these functional groups using selected biocatalytic reactions and produce a library of derivative compounds to be screened for biological activity. AZT contains four functional groups which are selected for biocatalytic modification: a primary hydroxyl, two carbonyls and a tertiary amine. The biosynthetic strategy is designated in the form of biocatalytic "reaction box" numbers which correspond to specific types of biocatalytic reactions acting on specific functional groups present in the starting compound. These "reaction boxes" are listed in Table III. The following biocatalytic "reaction boxes" are selected to synthesize an AZT derivative library: A3, A10, A11, C2, G6, G10 and G12. FIG. 1 illustrates the reaction of AZT with these selected biocatalytic reaction boxes.
c) The biocatalytic reaction boxes are entered into an automated system which is shown in FIG 2. The system is programmed to automatically execute the aforementioned biocatalytic reactions and synthesize a library of derivative products. A single automated system in capable of performing hundreds of pre-programmed biocatalytic reactions per day. We can estimate the total number of compounds that can be produced by analyzing the reaction products produced in each "reaction box" and multiplying the results. Table IV details the number of potential reaction products produced in each reaction box and the resulting total number of possible compounds produced. In the case of AZT, up to 1.75 x 10¹¹ new compounds can be synthesized. It should be pointed out that this compares very favorably to peptide libraries. For example, a library of hexapeptides will contain 20⁶ or 64 million compounds. This is a mere fraction, about 0.04% of the compounds that are possible using the biosynthetic approach described herein. Table V lists the results of a similar analysis on eleven other starting drug compounds. As shown in this table, the biocatalytic reactions can generate huge numbers of derivative compounds for drug screening.
d) The synthesized library of new compounds is assayed using enzyme inhibition assays, receptor- binding assays, immunoassays, and/or cellular assays to identify biologically active compounds. Before assaying the library of derivative compounds, any remaining AZT present in the library is either removed or inhibited to simplify the interpretation of screening assay results. This is easily accomplished by HPLC, TLC, or the addition of a monoclonal antibody specific for the starting compound. Numerous in vitro assays are available that test for anti-viral, anti-cancer, antihypertensive and other well known pharmacological activities. Some of these assays are listed in Table VI. Most of these assays are also performed on the automated system.
e) Libraries which test positive are further analyzed using a biocatalytic tracking protocol which quickly identifies the specific sequence of reactions responsible for the synthesis of the compound testing positive in the screening assay. The high degree of specificity exhibited by biocatalysts, i.e., the ability of a given enzyme to react with a given functional group, enables this approach to be easily performed. The library is characterized by the series of biocatalytic reactions used to produce it, a so called "biosynthetic history". Portions of the library are screened for biological activity until the specific reaction sequence producing the active compound is identified. FIG. 3 illustrates this tracking process. For example, the dark line path 15 illustrates the reaction pathway to the most active compound. The reaction sequence is repeated to produce a sufficient amount of product for chemical analysis. The specificity of the biocatalytic reactions also permits the accurate duplication of the reaction pathway producing the active compounds. The structure of the active compound is qualitatively determined by analyzing the starting compounds, substrates and identified biocatalytic reaction sequence. The structure is then confirmed using gas chromotography, mass spectroscopy, NMR spectroscopy and other organic analytical methods. This mode of identification eliminates the need for product purification and also reduces the amount of test screening required to identify a promising new drug compound. This process dramatically reduces the time necessary to synthesize and identify new drug compounds. In addition, this mode of active compound identification does not require immobilization technologies, and compounds can be synthesized and tested free in solution under in vivo like conditions using virtually any type of screening assay (receptor, enzyme inhibition, immunoassay, cellular, animal model).

Those skilled in the pharmaceutical arts will recognize the large number of biocatalytic conversions such as those listed in Table II and Table m, as well as the in vitro drug screening assays listed in Table VI.

Those skilled in the pharmaceutical arts will recognize that biocatalytic reactions are optimized by controlling or adjusting such factors as solvent, buffer, pH, ionic strength, reagent concentration and temperature.

The biocatalysts used in the biocatalytic reactions may be crude or purified enzymes, cellular lysate preparations, partially purified lysate preparations, living cells or intact non-living cells, used in solution, in suspension, or immobilized on magnetic or non-magnetic surfaces.

In addition, non-specific chemical reactions may also be used in conjunction with the biocatalytic reaction to obtain the library of modified starting compounds. Examples of such non-specific chemical reactions include: hydroxylation of aromatics and aliphatics; oxidation reactions; reduction reactions; hydration reactions; dehydration reactions; hydrolysis reactions; acid/based catalyzed esterification; transesterification; aldol condensation; reductive aminatian; amminolysis; dehydrohalogenation; halogenation; acylation; acyl substitution; aromatic substitution; Grignard synthesis; Friedel-Crafts acylation; etherification.

The biocatalytic reaction can be performed with a biocatalyst immobilized to magnetic particles forming a magnetic biocatalyst. The method of this embodiment is performed by initiating the biocatalytic reaction by combining the immobilized biocatalyst with substrate(s), cofactors(s) and solvent/buffer conditions used for a specific biocatalytic reaction. The magnetic biocatalyst is removed from the biocatalytic reaction mixture to terminate the biocatalytic reaction. This is accomplished by applying an external magnetic field causing the magnetic particles with the immobilized biocatalyst to be attracted to and concentrate at the source of the magnetic field, thus effectively separating the magnetic biocatalyst from the bulk of the biocatalyst reaction mixture. This allows for the transferral of the reaction mixture minus the magnetic biocatalyst from a first reaction vessel to a second reaction vessel, leaving the magnetic biocatalyst in the first reaction vessel. A second biocatalytic reaction is conducted completely independent of the first biocatalytic reaction, by adding a second biocatalyst immobilized to magnetic particles to the second reaction vessel containing the biocatalytic reaction mixture transferred from the first reaction vessel. Finally, these steps are repeated to accomplish a sequential series of distinct and independent biocatalytic reactions, producing a corresponding series of modified starting compounds.

The biocatalytic reactions can also be performed using biocatalysts immobilized on any surface which provides for the convenient addition and removal of biocatalyst from the biocatalytic reaction mixture thus accomplishing a sequential series of distinct and independent biocatalytic reactions producing a series of modified starting compounds.

The biocatalytic reactions can also be used to derivatize known drug compounds producing new derivatives of the drug compound and select individual compounds within this library that exhibit optimal activity. This is accomplished by the integration of a high affinity receptor into the biocatalytic reaction mixture, which is possible because of the compatibility of the reaction conditions used in biosynthesis and screening. The high affinity receptor is added to the reaction mixture at approximately one half the molar concentration of the starting active compound, resulting in essentially all of the receptor being bound with the starting active compound and an equal molar concentration of starting active compound free in solution and available for biocatalytic modification. If the biocatalytic reaction mixture produces a derivative which possesses a higher binding affinity for the receptor, which can translate into improved pharmacological performance, this derivative will displace the bound starting active compound and remain complexed with the receptor, and thus be protected from further biocatalytic conversions. At the end of the experiment, the receptor complex is isolated, dissociated and the bound compound analyzed. This approach accomplishes the identification of an improved version of the drug compound without the need to purify and test each compound individually.

The biocatalytic reactions and in vitro screening assays can be performed with the use of an automated robotic device. The automated robotic device having:
(a) an XY table with an attached XYZ pipetting boom to add volumetric amounts of enzyme, substrate, cofactor, solvent solutions and assay reagents from reagent vessels positioned on the XY table to reaction and assay vessels positioned on the same XY table;
(b) an XYZ reaction-vessel transfer boom attached to the same XY table used to transfer reaction and assay vessels positioned on the XY table to different locations on the XY table;
(c) a temperature incubation block attached to the same XY table to house the reaction and assay vessels during reaction incubations and control the temperature of the reaction mixtures;
(d) a magnetic separation block attached to the same XY table to separate the biocatalyst immobilized to magnetic particles from the biocatalytic mixture by applying an external magnetic field causing the magnetic particles to be attracted to and concentrate at the source of the magnetic filed, thus effectively separating them from the bulk of the biocatalytic reaction mixture; and
(e) a programmable microprocessor interfaced to the XYZ pipetting boom, and XYZ reaction-vessel transfer boom, the temperature block and the magnetic separation block to precisely control and regulate all movements and operations of these functional units in performing biocatalytic reactions to produce modified starting compounds and assays to determine desired activities.

Figure 2 illustrates the automated robotic device of this invention. Mounted in the frame 1 of the system are containers for starting compounds 2, and containers for reagents 3 such as enzymes, cofactors, and buffers. There are specific biosynthesis boxes 4 which contain reagents for various classes of reactions. The frame also has arrays of reaction vessels 5, and a heating block 6 with wells 7 for conducting reactions at a specific temperature. The frame has an area 8 for reagents for screening test 8 which contains reagents used for conducting screening tests, and area 9 which contains assay vessels for conducting screening tests, the automated system uses a X-Y-Z pipetting and vessel transfer boom 10 to dispense all reagents and solutions, and transfer reaction vessels.

In operation the X-Y-Z reaction-vessels transfer boom can deliver starting compounds and reagents to specific locations for making specific modified starting compounds which in turn can be delivered to specific locations for conducting assays. In this way the process of making modified starting compounds and testing for optimum activity is largely automated.

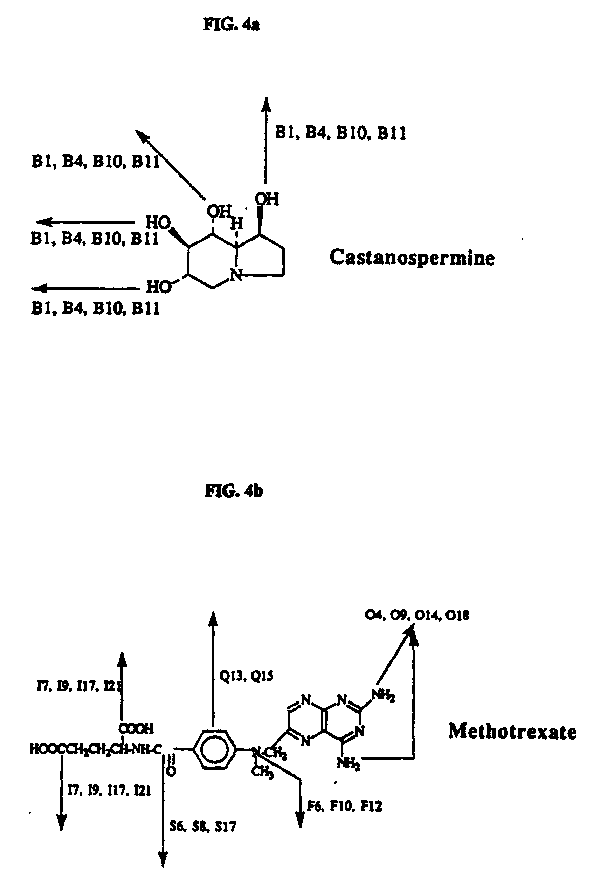

Figures 4a and 4b illustrate derivatization of castanospermine and methotrexate. All of these embodiments utilize the biocatalytic conversions set out in Table II and the assays set out in Table VI.

While the invention as described herein is directed to the development of drugs, those skilled in the biological arts will recognize that the methods of this invention are equally applicable to other biologically active compounds such as food additives, pesticides, herbicides, and plant and animal growth hormones.

**TABLE I.**

| **Major Functional Groups Available for Biocatalytic Modification*** | |
|---|---|
| A. | **Hydroxyl Groups** -- These groups can undergo numerous reactions including oxidation to aldehydes or ketones (1.1), acylation with ester donors (2.3, 3.1), glycosidic bond formation (2.4, 3.2, 5.3), and etherification (2.1, 3.3). Potential for stereo- and regio- selective synthesis as well as prochiral specificity. |
| B. | **Aldehydes and Ketones** -- These groups can undergo selective reduction to alcohols (1.1). This may then be followed by modifications of hydroxyl groups. |
| C. | **Amino Groups** These groups can undergo oxidative deamination (1.4), N-dealkylation (1.5, 1.11), transferred to other compounds (2.6), peptide bond synthesis (3.4, 6,3), and acylation with ester donors (2.3, 3.1). |
| D. | **Carboxyl Groups** -- These groups can be decarboxylated (1.2, 1.5, 4.1), and esterified (3.1, 3.6). |
| E. | **Thiol Groups** -- These groups can undergo reactions similar to hydroxyls, such as thioester formation (2.8, 3.1), thiol oxidation (1.8), and disulfide formation (1.8). |
| F. | **Aromatic Groups** -- These groups can hydroxylated (1.11, 1.13, 1.14), and oxidatively cleaved to diacids (1.14). |
| G. | **Carbohydrate Groups** -- These groups can be transferred to hydroxyls and phenols (2.4, 3.2, 5.3), and to other carbohydrates (2.4). |
| H. I. | **Ester and Peptide Groups** - These groups can be hydrolyzed (3.1, 3.4, 3.5, 3.6, 3.9), and transesterified (or interesterified) (3.1, 3.4). **Sulfate and Phosphate Groups** -- These groups can be hydrolyzed (3.1, 3.(, transferred to other compounds (2.7, 2.8), and esterified (3.1). |
| J. | **Halogens** -- These groups can be oxidatively or hydrolytically removed (1.11, 3.8), and added (1.11). |
| K. | **Aromatic Amines and Phenols --** These groups can be acylated (2.3, 3.1) or oxidatively polymerized (1.10, 1.11, 1.14). |

| | |
|---|---|
| *Numbers in parentheses correspond to the EC (Enzyme Commission) categorization of enzymes and enzyme classes. | |

**TABLE II.**

| **A Representative Subset of Biocatalytic Reactions Which May Be Used to Modify Functional Groups** | | |
|---|---|---|
| 1. | Oxidation of primary and secondary alcohols; Reduction of aldehydes and ketones. | |
| | | |
| | Reaction Boxes: A1, B1, C2, D2 | |
| | | |
| | Enzyme Class: 1.1. Dehydrogenases, Dehydtratases, Oxidases | |
| | | |
| | Representative Enzymes: | |
| | | Alcohol dehydrogenase |
| | | Glycerol Dehydrogenase |
| | | Glycerol-3-Phosphate Dehydrogenase |
| | | Xylulose Reductase |
| | | Polyol Dehydrogenase |
| | | Sorbitol Dehydrogenase |
| | | Glyoxylate Reductase |
| | | Lactate Dehydrogenase |
| | | Glycerate Dehydrogenase |
| | | β-Hydroxybutyrate Dehydrogenase |
| | | Malate Dehydrogenase |
| | | Glucose Dehydrogenase |
| | | Glucose-6-Phosphate Dehydrogenase |
| | | 3α- and 3β-Hydroxysteroid Dehydrogenase |
| | | 3α-, 20β-Hydroxsteroid Dehydrogenase |
| | | Fucose Dehydrogenase |
| | | Cytochrome-Dependent Lactate Dehydrogenase |
| | | Galactose Oxidase |
| | | Glucose Oxidase |
| | | Cholesterol Oxidase |
| | | Alcohol Oxidase |
| | | Glycolate Oxidase |
| | | Xanthine Oxidase |
| | | Fructose Dehydrogenase |
| | | |
| | Cosubstrates/Cofactors: NAD(P)(H) | |
| 2. | Acylation of primary and secondary alcohols. | |
| | | |
| | Reaction Boxes: A3, B4 | |
| | | |
| | Enzyme Classes: 3.1, 3.4, 3.5, 3.6 | |
| | | |
| | Representative Enzymes: Esterases, lipases, proteases, sulfatases, phosphatases, acylases, lactamases, nucleases, acyl transferases | |
| | | Esterases |
| | | Lipases |
| | | Phospholipase A |
| | | Acetylesterase |
| | | Acetyl Cholinesterase |
| | | Butyryl Cholinesterase |
| | | Pectinesterase |
| | | Cholesterol Esterase |
| | | Glyoxalase II |
| | | Alkaline Phosphatase |
| | | Acid Phosphatase |
| | | A Variety of nucleases |
| | | Glucose-6-Phosphatase |
| | | Fructose 1,6-Diphosphatase |
| | | Ribonuclease |
| | | Deoxyribonuclease |
| | | Sulfatase |
| | | Chondro-4-Sulfatase |
| | | Chondro-6-Sulfatase |
| | | Leucine Aminopeptidase |
| | | Carboxypeptidase A |
| | | Carboxypeptidase B |
| | | Carboxypeptidase Y |
| | | Carboxypeptidase W |
| | | Prolidase |
| | | Cathepsin C |
| | | Chymotrypsin |
| | | Trypsin |
| | | Elastase |
| | | Subtilisin |
| | | Papain |
| | | Pepsin |
| | | Ficin |
| | | Bromelain |
| | | Rennin |
| | | Proteinase A |
| | | Collagenase |
| | | Urokinase |
| | | Asparaginase |
| | | Glutaminase |
| | | Urease |
| | | Acylase I |
| | | Penicillinase |
| | | Cephalosporinase |
| | | Creatininase |
| | | Guanase |
| | | Adenosine Deaminase |
| | | Creatine Deaminase |
| | | Inorganic Pyrophosphatase |
| | | ATPase |
| | | Choline Acetyltransferase |
| | | Carnitine Acetyltransferase |
| | | Phosphotransacetylase |
| | | Chloramphenicol Acetyltransferase |
| | | Transglutaminase |
| | | γ-Glutamyl Transpeptidase |
| | Cosubstrates/Cofactors: Esters of alkyl, aryl, charged, polar/neutral groups. These acyl donors can be chosen from the class consisting of the following structural formulae: | |
| | | |
| | R-O-CO-R' | |
| | | |
| | Where R = alkyl, vinyl, isopropenyl haloalkyl, aryl, derivatives of aryl (i.e., nitrophenyl) and R' can be any alkyl or aryl group with or without derivatives. Such derivatives include halogens, charged functional groups (i.e., acids, sulfates, phosphates, amines, etc.), glycols (protected or unprotected), etc. | |
| | | |
| 3. | Transglycosylation of primary and secondary alcohols. | |
| | | |
| | Reaction Boxes: A10,B10 | |
| | | |
| | Enzyme Class: 2.4, 3.2 | |
| | | |
| | Representative Enzymes: | |
| | | Phosphorylase a |
| | | Phosphorylase b |
| | | Dextransucrase |
| | | Levansucrase |
| | | Sucrose Phosphorylase |
| | | Glycogen Synthase |
| | | UDP-Glucuronyltransferase |
| | | Galactosyl Transferase |
| | | Nucleoside Phosphorylase |
| | | α- and β-Amylase |
| | | Amyloglucosidase (Glucoamylase) |
| | | Cellulase |
| | | Dextranase |
| | | Chitinase |
| | | Pectinase |
| | | Lysozyme |
| | | Neuraminidase |
| | | Xylanase |
| | | α- and β-Glucosidase |
| | | α- and β-Galactosidase |
| | | α- and β-Mannosidase |
| | | Invertase |
| | | Trahalase |
| | | β-N-Acetylglucosaminidase |
| | | γ-Glucuronidase |
| | | Hyaluronidase |
| | | β-Xylosidase |
| | | Hesperidinase |
| | | Pullulanase |
| | | α-Fucosidase |
| | | Agarase |
| | | Endoglycosidase F |
| | | NADase |
| | | Glycopeptidase F |
| | | Thioglucosidase |
| | | |
| | Cosubstrates/Cofactors: All available sugars and their derivatives. These sugars can be monosaccharides, disaccharides, and oligosaccharides and their derivatives. | |
| | | |
| 4. | Etherification of primary and secondary alcohols | |
| | | |
| | Reaction Boxes: A11, B11 | |
| | | |
| | Enzvme Classes: 2.1, 3.2 | |
| | | |
| | Representative Enzymes: | |
| | | Catechol α-Methyltransferase |
| | | Aspartate Transcarbamylase |
| | | Ornithine Transcarbarnylase |
| | | S-Adenosylhomocysteine Hydrolase |
| | | |
| | Cosubstrates/Cofactors: Alcohols or ethers of any chain length. | |
| 5. | Acylation of primary and secondary amines. | |
| | | |
| | Reaction Boxes: E3, E4 | |
| | Enzyme Classes: 2.3, 3.1, 3.4, 3.5, 3.6 | |
| | | |
| | Representative Enzymes: | |
| | | Choline Acetyltransferase |
| | | Camitine Acetyltransferase |
| | | Phosphotransacetylase |
| | | Chloramphenicol Acetyltransferase |
| | | Transglutaminase |
| | | γ-Glutamyl Transpeptidase |
| | | Esterases |
| | | Lipases |
| | | Phospholipase A |
| | | Acetylesterase |
| | | Acetyl Cholinesterase |
| | | Butyryl Cholinesterase |
| | | Pectinesterase |
| | | Cholesterol Esterase |
| | | Glyoxylase II |
| | | Alkaline Phosphatase |
| | | Acid Phosphatse |
| | | A Variety of nucleases |
| | | Glucose-6-Phosphatase |
| | | Fructose 1,6-Diphosphatase |
| | | Ribonuclcase |
| | | Deoxyribonuclease |
| | | Sulfatase |
| | | Chondro-4-Sulfatase |
| | | Chondro-6-Sulfatase |
| | | Leucine Aminopeptidase |
| | | Carboxypeptidase A |
| | | Carboxypeptidase B |
| | | Carboxypeptidase Y |
| | | Carboxypeptidase W |
| | | Prolidase |
| | | Cathepsin C |
| | | Chymotrypsin |
| | | Trypsin |
| | | Elastase |
| | | Subtilisin |
| | | Papain |
| | | Pepsin |
| | | Ficin |
| | | Bromelin |
| | | Rennin |
| | | Proteinase A |
| | | Collagenase |
| | | Urokinase |
| | | Asparaginase |
| | | Glutaminase |
| | | Urease |
| | | Acylase I |
| | | Pinicillinase |
| | | Cephalosporinase |
| | | Creatininase |
| | | Guanase |
| | | Adenosine Deaminase |
| | | Creatine Deaminase |
| | | Inorganic Pyrophosphatase |
| | | ATPase |
| | | |
| | Cosubstrates/Cofactors: See example number 2, above. | |
| | | |
| 6. | Esterification of carboxylic acids. | |
| | | |
| | | Reaction Boxes: 17. |
| | | |
| | | Enzyme Classes: 3.1, 3.6 |
| | Representative Enzymes: | |
| | | Esterases |
| | | Lipases |
| | | Phospholipase A |
| | | Acetylesterase |
| | | Acetyl Cholinesterase |
| | | Butyryl Cholinesterase |
| | | Pectinesterase |
| | | Cholesterol Esterase |
| | | Glyoxylase II |
| | | Alkaline Phosphatase |
| | | Acid Phosphatase |
| | | A Variety of nucleases |
| | | Glucose-6-Phosphatase |
| | | Fructose 1,6-Diphosphatase |
| | | Ribonuclease |
| | | Deoxyribonuclease |
| | | Sulfatase |
| | | Chondro-4-Sulfatase |
| | | Chondro-6-Sulfatase |
| | | Inorganic Pyrophosphatase |
| | | APTase |
| | | |
| | Cosubstrates/Cofactors: alcohols of any chain length being alkyl, aryl, or their structural derivatives. | |

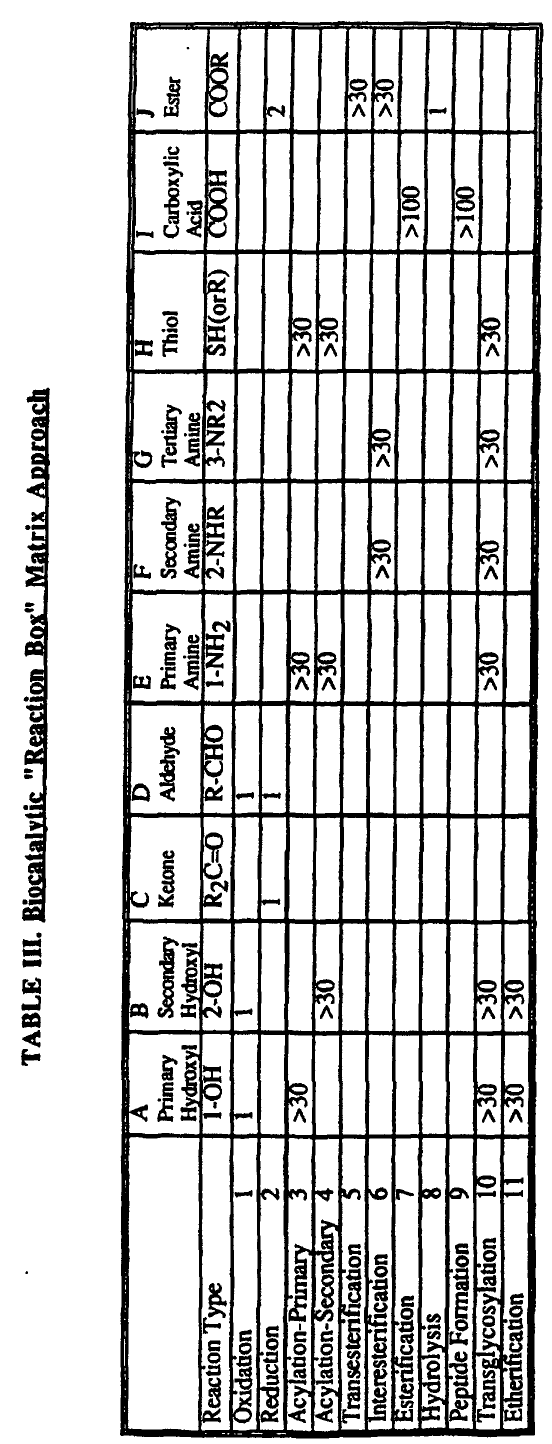

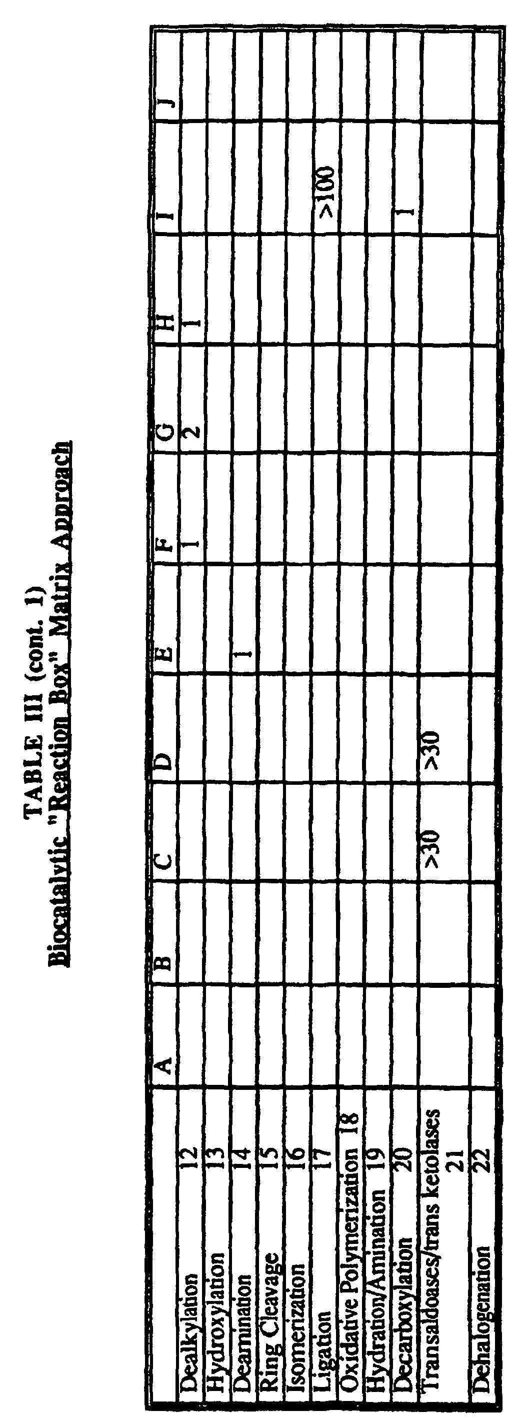

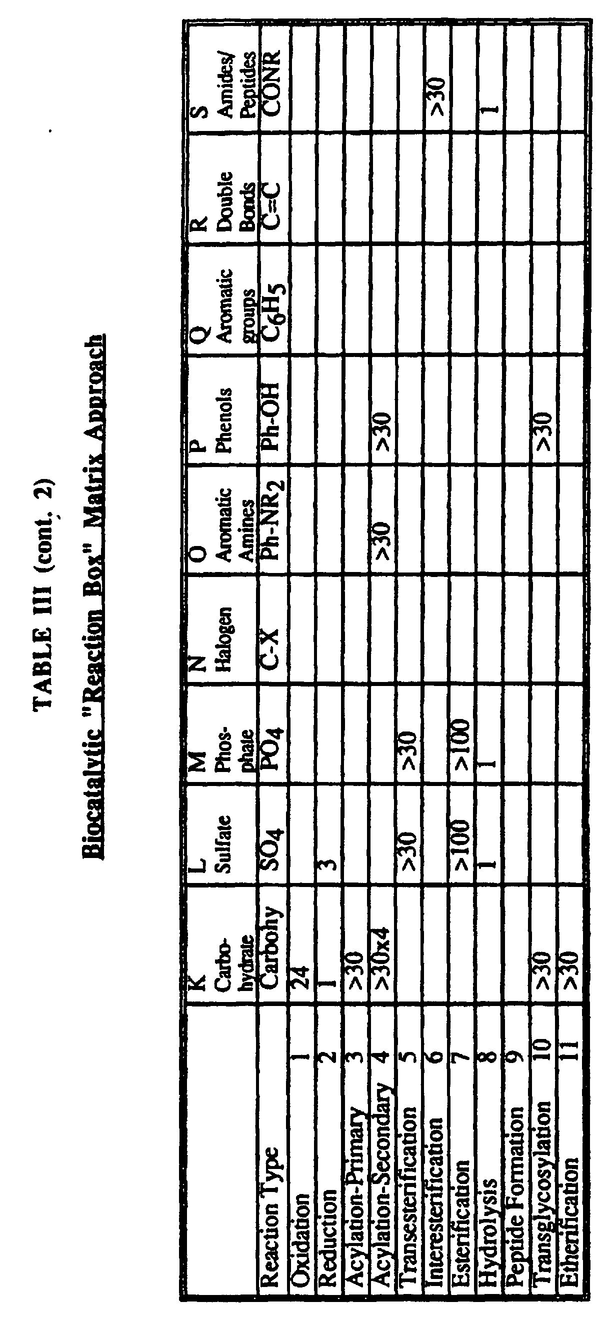

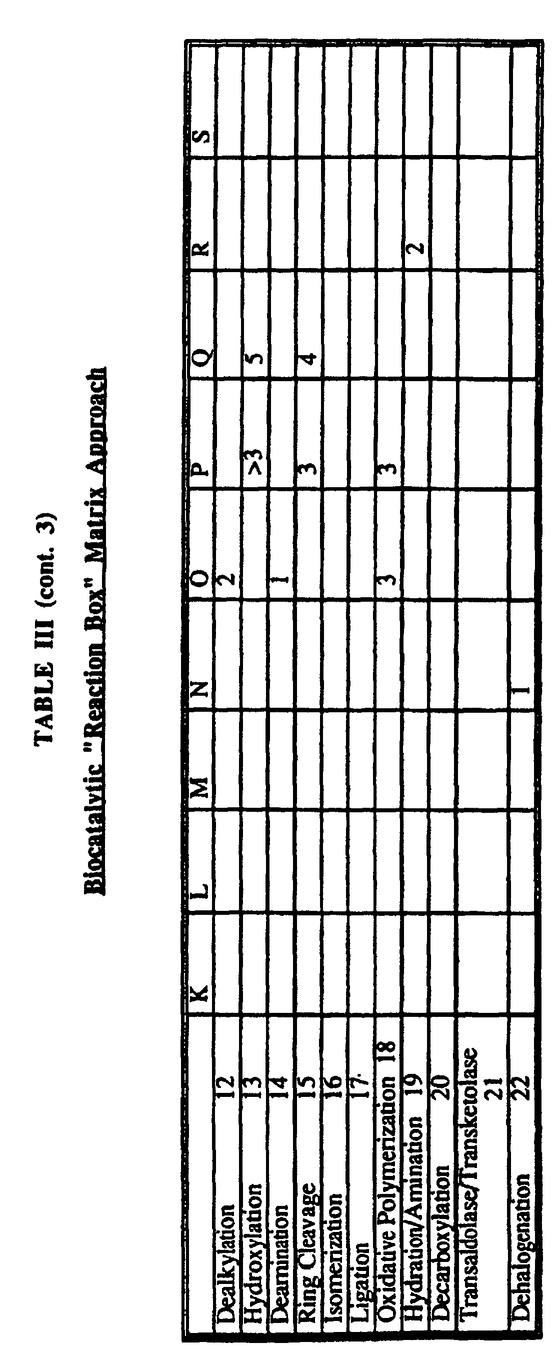

**TABLE IV.**

| **Reaction Box Analysis of AZT Derivatization Indicating the Total Number of Possible Reaction Products** | |
|---|---|
| Reaction Box | Number of Possible Products |
| A3 | 30^{(a)} |
| A10 | 30^{(b)} |
| All | 30 |
| C2 | 2 x 30^{(c)} |
| C2 | 2 x 30^{(c)} |
| G6 | 30 |
| G10 | 30 |
| G12 | x2 |
| Total | 1.75 x 10¹¹ distinct compounds(d) |

| | |
|---|---|
| (a) Assuming 30 different acyl donors to be added to this reaction mixture. This includes alkyl, aryl, and of different lengths, | |
| (b) Assuming 30 UDP-sugars used in this reactin box, | |
| (c) Reduction of the ketones to secondary alcohols leads to the potential acylation of the secondary alcohols and adds 30-fold more potential products; and (d) Each box's possible permutations are multiplied together to estimate the total number of compounds synthesized. | |

**TABLE V.**

| **Reaction Box Analysis of Established Drugs Indicating the Total Number of Possible Reaction Products** | | |
|---|---|---|
| Starting Compound | Number of Functional Groups | Estimated Number of Derivatives |
| Castanospermine | 4 | 810,000 |
| Cyclosporin | 24 | billions |
| Gentamicin | 8 | billions |
| Haloperidol | 3 | 120 |
| Methotrexate | 7 | greater than 10¹⁹ |
| Muscarine | 2 | 2,400 |
| Prazosin | 6 | 288,000 |
| Prednisone | 12 | 46,080,000 |
| Thyroxine | 3 | 2,160,000 |
| Valproic Acid | 1 | 900 |
| Vancomycin | many | billion |

**TABLE VI.**

| **Representative Subset of Screening Assays Possible to Test for Anti-Cancer, Anti-Viral and Anti-Hypertensive Activities** | |
|---|---|
| | **Anti-Cancer Drugs** |
| 1. | KB (Eagle) cell culture assay |
| 2. | Inhibition of the growth of human breast cancer cell lines *in vitro* |
| 3. | Inhibition of the growth of P388 leukemia cells *in vitro* |
| 4. | Inhibition of the growth of murine L1210 cells *in vitro* |
| 5. | Inhibition of gylcinamide ribonucleotide formyltransferase activity |
| 6. | Inhibition of ribonucleotide reductase activity |
| 7. | Inhibition of protein kinase C activity |
| 8. | Inhibition of human aromatase activity |
| 9. | Inhibition of DNA topoisomerase II activity |
| 10. | Inhibition of dihydrofolate reductase |
| 11. | Inhibition of aminoimidazole carboxamide ribonucleotide formyltransferase |

| | **Anti-AIDS Drugs** |
|---|---|
| 1. | Inhibition of HIV virus replication devoid of cytotoxic activity |
| 2. | Inhibition of HIV protease activity |
| 3. | Soluble-formazan assay for HIV-1 |
| 4. | Inhibition of HIV reverse transcriptase activity |

| | **Anti-Hypertensive Drugs** |
|---|---|
| 1. | Inhibition of ACE activity |
| 2. | Inhibition of human plasma renin |
| 3. | Inhibition of in vitro human renin |
| 4. | Inhibition of angiotensin converting enzyme |
| 5. | Alpha 1-adrenergic receptor binding assay |
| 6. | Alpha 2-adrenergic receptor binding assay |
| 7. | Beta-adrenergic receptor binding assay (bronchodilator, cardiotonic, tocolytic, anti-anginal, anti-arrhythmic, anti-glaucoma) |
| 8. | Dopamine receptor binding assay (anti-migraine, anti-parkinsonian, anti-emetic, anti-psychotic) |

### Example 1

### 1. Enzymatic esterification of taxol

### 1.1 Analysis of reaction products

Products of enzymatic esterification of taxol were analyzed using reversed-phase HPLC on a Waters system with a 990 photodiode array detector, and a 3.9 x 300 mm µBondapak C₁₈ column. The solvent system used consisted of a water:acetonitrile mixture (40:15 ν/ν) and isopropanol. Elution was performed at 1 ml/min according to the following linear gradient program:

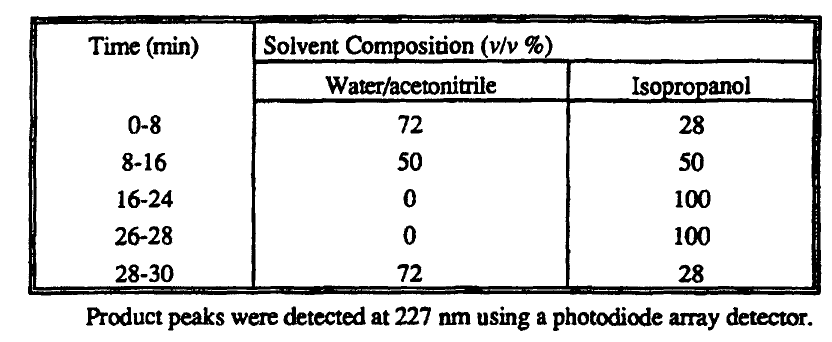

### 1.2 Enzymatic synthesis of taxol esters - Identification of active biocatalysts (Reaction Box B-4)

A mixture of 55 enzymes (Table VII) consisting of crude lipases, crude proteases, and purified proteases (total mass of 600 mg) was added to a solution of taxol (17.0 mg, 20 µmol) and vinyl butyrate (0.25 ml, 1.5 mmol) in 2.9 ml *tert* amyl alcohol. The mixture was placed into a septum-sealed glass vial, sonicated for 30 s, and put into an orbit shaker operating at 250 rpm and 35°C for a period of 48 h. The reaction was then stopped by removing the suspended solid catalyst by centrifugation. The supernatant containing taxol esters was evaporated to dryness in vacuum and redissolved in 0.3 ml methanol. An aliquot of the resultant concentrated solution (15 µl) was diluted with 250 µl methanol and analyzed by HPLC as described in 1.1. The chromatogram revealed a peak with a retention time of 9.7 min (unreacted taxol), and a peak at 14.6 min (2'-taxol butyrate, yield 7%).

The enzymes were then split into three groups: 26 lipases (100 mg/ml), 26 crude proteases (100 mg/ml), and 4 purified proteases (10 mg/ml) and the identical reaction as above performed. Only the purified proteases showed significant activity with a yield of 2'-butyrate of 15% after 48 h. The purified proteases were then divided into individual enzymes (all used at a concentration of 10 mg/ml) and thermolysin (a bacterial protease from *Bacillus thermoproteolyticus rokko a.k.a.* thermolysin) was identified as the most active enzyme with a yield after 48 h of 24%. This approach demonstrates that active biocatalysts can be easily identified by a sequential process of eliminating unreactive biocatalysts.

**TABLE VII.**

| **Enzymes used in the acylation of taxol** | | | |
|---|---|---|---|
| **I.** | **Lipases and Esterases** | | |
| | Enzyme | Source | Company |
| | CES | | Amano |
| | L-10 | | Amano |
| | G | *Penicillium sp.* | Amano |
| | N | *Rhizopus niveus* | Amano |
| | AP | *Aspergillus niger* (acid stable) | Sigma |
| | Wheat Germ | | Amano |
| | GC-20 | *Geotrichwn candidum* | Amano |
| | AY-30 | *Candida rugosa* | Amano |
| | P | *Pseudomonas cepacia* | Amano |
| | AG-975 | | Sigma |
| | Porcine Pancreatic | | Amano |
| | APF | *A. niger* | Amano |
| | R-10 | | Amano |
| | AK | *Pseudomonas sp.* | Amano |
| | PGE | Calf tongue root | Amano |
| | D | | Amano |
| | GC-4 | | Amano |
| | CE | | Sigma |
| | *Candida rugosa* | | Amano |
| | FAP-15 | *Rhizopus sp.* | Amano |
| | MAP-10 | *Mucor sp.* | Amano |
| | Enzeco K16825 | | Enzeco |
| | Lamb Pre-Gastric Esterase | | Quest |
| | Calf Pre-Gastric Estese | | Quest |
| | Esterase 30,000 #3586 | | Gist Brocades |
| | Lipase 80,000 #5093 | *Rhizopus sp.* | Gist Brocades |

| **II.** | **Proteases** | | |
|---|---|---|---|
| | Enzyme | Source | Company |
| | B | *Penicillium sp.* | Amano |
| | Papain | Papaya | Sigma |
| | 2A | *Aspergillus oryzae* | Amano |
| | Proleather | *Bacillus sp.* | Amano |
| | N | *B. subtilis* (neutral) | Amano |
| | Acid Stable | *Rhizopus sp.* | Amano |
| | Alcalase-2T | *B. Licheniformis* (Subtilisin Carlsberg) | Novo |
| | Bromelain | Pineapple | Sigma |
| | Alkaline Protease | | Quest |
| | Fungal Protease #9240810 | | Biocon |
| | Acid Protease #8221108 | | Biocon |
| | Neutral Protease 900,000 | | Biocon |
| | #6W16B | | |
| | HT | | MKC |
| | Rapidase S-90 | *B. subtilis* | Gist Brocades |
| | HT-Proteolytic 200 | | Solvay |
| | Opticlean M-375 | Alkaline protease (from *Bacillus sp)*. | Solvay |
| | Optimase M-440 | Alkaline protease (from *Bacillus sp).* | Solvay |
| | Fungal Protease 60,000 | | Solvay |
| | Esperase-4T | Alkaline protease (from *Bacillus sp*). | Novo |
| | Peptidase A | *A. oryzae* | Amano |
| | Prozyme 6 | *Aspergillus sp.* | Amano |
| | Protease M | *A. oryzae* | Amano |
| | Newlase A | *A. niger* (acid stable) | Amano |
| | Acylase Conc. | *Aspergillus sp.* | Amano |
| | Protease S | *Bacillus sp.* (thermostable) | Amano |
| | Acylase I | Porcine kidney | Sigma |
| | | | |

| **III.** | **Purified Enzymes** | | |
|---|---|---|---|
| | Enzyme | Source | Company |
| | Trypsin | Bovine pancreas | Sigma |
| | Chymotrypsin | Bovine pancreas | Sigma |
| | Subtilisin Carlsberg | *Bacillus liquefaciens* | Sigma |
| | Protease X | *B. thermoproteolyticus rokko* | Sigma |

### 1.3. Enzymatic synthesis of a mixture of taxol esters - Sequential reaction box derivatization of taxol

The optimal enzyme catalyst used for the synthesis was produced by freeze-drying an aqueous solution containing thermolysin, KCl and potassium phosphate buffer adjusted to pH 7.5. The solid catalyst obtained after free-drying contained 5% enzyme, 94% KCl and 1% potassium phosphate. The powdered enzyme catalyst (335 mg - containing 16.75 mg thermolysin) was added to a solution of taxol (17.0 mg, 20 µmol) and vinyl caproate (straight-chain C₆ ester) (0.24 ml. 1.5 mmol) in 2.9 ml tert-amyl alcohol. The mixture was placed into a septum-sealed glass vial, sonicated for 30 s, and put into an orbit shaker operating at 250 rpm and 35°C. After 28 h the reaction mixture (including enzyme) was removed and the full contents of the mixture added to a separate solution containing vinyl propionate (0.24 ml, 2.2 mmol), vinyl acrylate (0.24 ml, 2.2 mmol), and vinyl butyrate (0.24 ml, 1.9 mmol). This second reaction was placed on the shaker and incubated at 250 rpm at 35°C. After 24 h, the reaction mixture (including enzyme) was removed and the full contents of the mixture added to a separate solution containing vinyl acetate (0.25 ml, 2.6 mmol) and vinyl chloroacetate (0.24 ml, 1.8 mmol). This reaction was allowed to proceed for 24 h at 250 rpm and 35°C. The sequential reaction was then stopped by removing the suspended solid catalyst by centrifugation. The sequential reaction was aided by the soluble nature of the taxol and taxol derivatives. The supernatant containing taxol esters was evaporated to dryness in vacuum and redissolved in 0.3 ml methanol. An aliquot of the resultant concentrated solution (15 µl) was diluted with 250 µl methanol and analyzed by HPLC as described in 1.1. The chromatogram revealed a peak with retention time 9.7 min (unreacted taxol), a broad peak between 11 and 13 min (total estimated yield 52%), peaks at 14.6 min (yield 9%) and 17.4 min (yield 18%), and a small peak at 18.4 min (yield 0.2%). Total reaction yield was approximately 80%.

### 1.4 Removal of taxol from reaction mixture

A concentrated methanol solution of reaction products produced as described in 1.3 was applied on a preparative TLC silica plate (Whatman, 20x20 cm, silica layer thickness 500 µm, containing fluorescent marker), and the plates were developed using a solvent mixture of chloroform:acetonitrile (4:1 ν/ν). Positions of product spots were determined by irradiating the plates with ultraviolet light. The R_{f} value of taxol is 0.16 and the R_{f} of the taxol esters range from 0.28 to 0.71. The products were removed from the TLC plate and dissolved in ethyl acetate. The products were then dried *in vacuo*.

### 1.5 Screening the mixture of taxol esters

The library of taxol derivatives described above was screened for cytotoxicity against HL-60 cells, a promyelocytic leukemia cell line, and MOLT-4 cells, a lymphoblastic leukemia cell line. Cells were seeded in 96-well plates at densities of 30,000 cells/well and grown in RPMI-1640 medium containing 10% bovine fetal calf serum at 37°C for 24 h. The medium was then replaced with fresh medium containing the taxol derivatives (excluding taxol, which had been removed by preparative thin-layer chromatography) dissolved in DMSO at final concentrations ranging from 100 nM to 0.1 nM. The final concentration of DMSO in the cell medium was 0.5% (ν/ν). After 72 h, samples were removed for cell counts. Total cell number and viability were determined by trypan blue exclusion and manual cell counting on a hemacytometer. The dose-response data are reported in Table IX. The hemacytometer cell counts revealed that the taxol-derivative library contained at least one cytotoxic derivative.

### 1.6 Backtracking to identify active product(s)

### 1.6.1 Enzymatic synthesis of individual taxol esters - Identification of active taxol esters by building-up the reactions comprising taxol acylation in 1.3 above.

Given the predictable nature of the enzymatic acylation reactions, it is possible to identify the possible products without analyzing the chemical compositions of the mixture. The possible products were, therefore, synthesized individually, as described below, and screens on these individual ester products were then initiated as part of the backtracking process.

The powdered enzyme catalyst prepared as described in 1.3 above (140 mg) was added to a solution of taxol (5.5 mg, 6.5 µmol) and on individual vinyl ester (80 µl, approximately 2 mmol) in 1.0 ml tert-amyl alcohol. The following vinyl esters were used as acylating agents: acetate, chloroacetate, acrylate, propionate, butyrate, and caproate. Each mixture was placed into a septum-sealed glass vial, sonicated for 30 s, and put into an orbit shaker operating at 250 rpm and 35°C. After 96 h the reaction was stopped by removing the suspended solid catalyst by centrifugation. Supernatants containing taxol esters were evaporated to dryness *in vacuo* and redissolved in 0.3 ml methanol each. An aliquot of each resultant concentrated solutions (20 µl) was diluted with 80 µl methanol and analyzed by HPLC as described in 1.1. Results of HPLC analysis are given in Table VIII.

**Table VIII.**

| **Retention times (RT) and R**_{**f**} **values of Taxol esterification products*** | | | | | | | | | | |
|---|---|---|---|---|---|---|---|---|---|---|
| Ester | 2'-ester | | | 7-ester | | | 2',7-diester | | | Total yield (%) |
| | RT (min) | R_{f} | Yield (%) | RT (min) | R_{f} | Yield (%) | RT (min) | R_{f} | Yield (%) | |
| Acetate | 10.5 | 0.28 | 57 | 13.1 | 0.48 | 30 | 15.2 | 0.57 | 2 | 89 |
| Acrylate | 12.2 | 0.39 | 80 | 14.1 | 0.56 | 10 | 15.6 | 0.67 | 0.4 | 90 |
| Chloroacetate | 12.5 | 0.32 | 70 | 15.9 | 0.51 | 3 | 0.71 | 0.71 | 12 | 85 |
| Propionate | 12.8 | 0.39 | 78 | 14.5 | 0.58 | 12 | - | - | - | 90 |
| Butyrate | 14.5 | 0.41 | 67 | 15.8 | 0.61 | 11 | - | - | - | 78 |
| Hexanoate | 17.3 | 0.47 | 50 | 18.1 | 0.67 | 7 | - | - | - | 57 |

| | | | | | | | | | | |
|---|---|---|---|---|---|---|---|---|---|---|
| *Taxol: retention time 9.7 min. R_{f} 0.16. | | | | | | | | | | |

### 1.6.2. Recovery of reaction products by thin layer chromatography (TLC)

Individual ester reaction products were separated from taxol by TLC. Concentrated methanol solutions of reaction products produced as described in 1.6 were applied on preparative TLC silica plates (Whatman, 20x20 cm, silica layer thickness 500 µm, containing fluorescent market), and the plates were developed using a solvent mixture of chloroform:acetonitrile (4:1 ν/ν). Positions of product spots were determined by irradiating the plates with ultraviolet light. The R_{f} values of the products are given in Table VIII. Product spots were scraped off the plates separately and scrapings were eluted with 15 ml ethyl acetate to recover the product. Dry products were obtained by evaporation of ethyl acetate *in vacuo*.

### 1.6.3 Screening individual taxol esters

To determine which of the newly synthesized product(s) was active, the synthetic history of the library was "backtracked" by enzymatically synthesizing all possible ester products individually (as described above). Following isolation of the products by TLC, each derivative was tested for cytotoxic activity as described above. The cytotoxicity experiments revealed that two derivatives, 2'-chloroacetyltaxol and 2'-acryloyltaxol, are active against both cell lines (Table IX).

**Table IX.**

| **Percent Viability of MOLT-4 and HL-60 Cells** After 72 h exposure to taxol and its derivatives at various concentrations | | | | |
|---|---|---|---|---|
| | 100 nM | 10 nM | 1 nM | 0.1 nM |
| **MOLT-4** | | | | |
| Taxol | 1.22 | 11.1 | 92.8 | 92.9 |
| Library | 3.13 | 67.9 | 95.9 | 95.0 |
| 2'-Chloroacetyltaxol | 4.26 | 91.9 | 94.4 | 96.9 |
| 2'-Acryloyltaxol | 2.70 | 61.0 | 95.6 | 91.0 |

| **HL-60** | | | | |
|---|---|---|---|---|
| Taxol | 0.00 | 32.6 | 96.1 | 98.3 |
| Library | 5.26 | 98.3 | 98.5 | 99.0 |
| 2'-Chloroacetyltaxol | 2.13 | 99.5 | 97.3 | 99.5 |
| 2'-Acryloyltaxol | 4.69 | 87.1 | 96.9 | 97.5 |

### Example 2

### 2. Enzymatic hydrolysis of taxol

### 2.1 Analysis of reaction products

Products of enzymatic hydrolysis of taxol were analyzed using reversed-phase HPLC on a Waters system with a 990 photodiode array detector, with 3.9 x 300 mm µBondapak C₁₈ column. The solvent system used consisted of water and acetonitrile. The solvents were fed into the HPLC system at 1 ml/min according to the following linear gradient program:

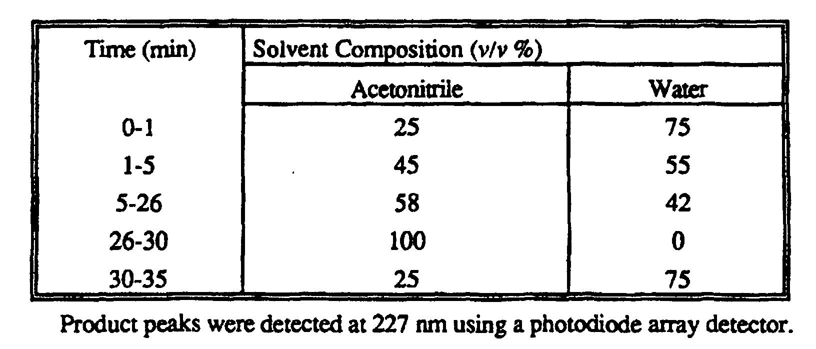

### 2.2 Enzymatic hydrolysis of taxol (Reaction Boxes J-8 and S-8)

The enzyme catalyst used for hydrolysis was produced by freeze-drying an aqueous solution (adjusted to pH 7.5) containing equal weight amounts of thermolysin, subtilisin Carlsberg, chymotrypsin and trypsin. The enzyme catalyst (1.1 mg) was dissolved in 0.7 ml 0.1 M potassium phosphate buffer pH 7.5. The aqueous enzyme solution was added to a solution of taxol (2.0 mg, 2.4 µmol) in 0.3 ml tert amyl alcohol. The biphasic reaction system produced in this way was placed into a septum-sealed glass vial and put on an orbit shaker operating at 75 strokes/min and 20°C. After 26 h the organic solvent layer was separated, evaporated to dryness and redissolved in 0.2 ml methanol for HPLC analysis. The aqueous phase was diluted with 3.5 ml methanol and insoluble solids (precipitated enzymes) were removed by centrifugation. Clear supernatant was evaporated into dryness and redissolved in 0.2 ml methanol for HPLC analysis. HPLC analysis was performed as described in 2.1. Neither taxol, nor any hydrolysis products were detected in the methanol solution obtained after workup of the aqueous phase of the biphasic reaction system. On the other hand, the chromatogram of the methanol solution of the dry residue obtained from the organic layer revealed a peak with retention time of 20.4 min (unreacted taxol) and a peak at 18.4 min, representing a product of enzymatic hydrolysis of taxol, as identified by the characteristic uv-absorbance scan. The estimated yield of this product was 9%.

### Example 3

### 3. Enzymatic glycosylation of taxol

### 3.1 Analysis of Reaction Products

Products of enzymatic glycosylation of taxol were analyzed using reversed-phase HPLC on a Waters system with a 990 photodiode detector, with a 3.9 x 300 mm µBondapak C₁₈ column. The solvent system used consisted of water and methanol. The solvents were fed into the HPLC system at 1 ml/min according to the following linear gradient program:

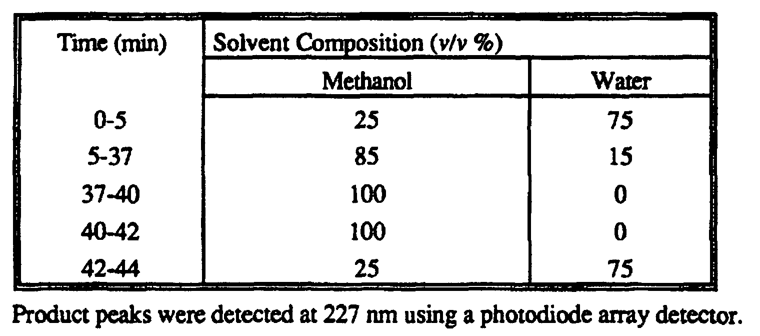

### 3.2 Enzymatic glycosylation of taxol

Glycosylation of taxol was performed using α-glucosidase from baker's yeast as a biocatalyst. For reference, the enzymes used were: α-glucosidase from brewer's yeast (pH 7.0), α-glucosidase from baker's yeast (pH 7.0), β-galactosidase from *E. coli* (pH 5.0), β-galactosidase from *A. oryzae* (pH 5.0), β-glucuronidase from bovine liver (pH 5.0). These enzymes were used in two mixtures (one at pH 7.0 and one at pH 5.0) and it was determined that only the mixture at pH 7.0 was active in glycosylating taxol. This mixture was then divided into individual enzymes where it was found that α-glucosidase from baker's yeast was the active biocatalyst.

## Claims

1. A method for generating a library of non biological organic compounds with a desired activity comprising:
(a) selecting a starting compound which exhibits drug activity or is believed to exhibit drug activity for a given disease or disorder;
(b) analyzing the starting compound with respect to its functional group content;
(c) adding to the starting compound at least one biocatalyst which is known to act on at least one selected functional group on the compound;
(d) selecting reaction conditions under which the at least one biocatalyst will react with the selected functional group on the compound to yield at least one modified compound;
(e) adding at least one second biocatalyst which is known to act on at least one selected functional group on the compound to the modified compound to produce a second population of modified compounds; and
(f) repeating the method to yield a library of modified compounds,
with the proviso that the starting compound is not a peptide or a protein when the biocatalyst is a protease.

2. A method according to claim 1, wherein the second biocatalyst is added to the modified compound separately from the biocatalyst of step (d).

3. A method according to claim 1, wherein the additional biocatalyst is added to the starting compound with the biocatalyst of step (d).

4. A method according to claim 1, further comprising :
(g) screening the modified compounds in the library for an activity.

5. A method according to claim 1, wherein the functional group on the starting compound is selected from the group consisting of hydroxyl groups, aldehyde groups, ketone groups, amino groups, carboxyl groups, thiol groups, aromatic groups, carbohydrate groups, ester groups, peptide groups, sulfate groups, phosphate groups, halogen groups, aromatic amine groups, and phenol groups.

6. A method according to claim 1, wherein the biocatalysts are selected from the group consisting of dehydrogenases, dehydratases, oxidases, esterases, lipases, proteases, sulfatases, phosphatases, acylases, lactamases, nucleases, acyl transferases, transglycosylases of primary and secondary alcohols, etherificases of primary and secondary alcohols, acylases of primary and secondary amines, and esterificases.

7. A method according to claim 1, which is automated.

8. A method according to claim 4, wherein the screening is automated.

9. A method according to claim 1, wherein the biocatalysts are in a formulation selected from the group consisting of crude enzymes, purified enzymes, cellular lysate preparations, partially purified lysate preparations, living cells, and intact non-living cells.

10. A method according to claim 1, wherein the compounds are reacted with the biocatalysts in a medium selected from the group consisting of solution, suspension, and immobilization means.

11. A method according to claim 10, wherein the biocatalyst is immobilized.

12. A method according to claim 11, wherein the biocatalyst is immobilized to a magnetic particle.

13. A method according to claim 1, further comprising reacting the compound using non-specific chemical reactions selected from the group consisting of hydroxylation, oxidation, reduction, dehydration, hydration, hydrolysis, esterification, transesterification, aldol condensation, reductive amination, ammonolysis, dehydrohalogenation, halogenation, acylation, acyl substitution, aromatic substitution, Grignard synthesis, and Friedel-Crafts acylation.

14. A method according to claim 1, further comprising adding to the reaction between the compound and at least one biocatalyst at least one cofactor for a biocatalyst.

15. A method according to claim 1, wherein the biocatalysts are selected from the group consisting of alcohol dehydrogenase, glycerol dehydrogenase, glycerol-3-phosphate dehydrogenase, xylulose reductase, polyol dehydrogenase, sorbitol dehydrogenase, glyoxylate reductase, lactate dehydrogenase, glycerate dehydrogenase, β-hydroxybutyrate dehydrogenase, malate dehydrogenase, glucose dehydrogenase, glucose-6-phosphate dehydrogenase, 3α- and 3β-hydroxysteroid dehydrogenase, 3α-, 20β-hydroxysteroid dehydrogenase, fucose dehydrogenase, cytochrome-dependent lactate dehydrogenase, galactose oxidase, glucose oxidase, cholesterol oxidase, alcohol oxidase, glycolate oxidase, xanthine oxidase, fructose dehydrogenase, phospholipase A, acetylesterase, acetyl cholinesterase, butyryl cholinesterase, pectinesterase, cholesterol esterase, glyoxalase II, alkaline phosphatase, acid phosphatase, glucose-6-phosphatase, fructose 1,6-diphosphatase, ribonuclease, deoxyribonuclease, chondro-4-sulfatase, chondro-6-Sulfatase, leucine aminopeptidase, carboxypeptidase A , carboxypeptidase B, carboxypeptidase Y, carboxypeptidase W, prolidase, cathepsin C, chymotrypsin, trypsin, elastase, subtilisin, papain, pepsin, ficin, bromclaim, rennin, proteinase A, collagenase, urokinase, asparaginase, glutaminase, urease, acylase I, pencillinase, cephalosporinase, creatininase, guanase, adenosine deaminase, creamine, deaminase, inorganic pyrophosphatase, ATPase, choline acetyltransferase, carnitine, acetyltransferase, phosphotransacetylase, chloramphenicol acetyltransferase, transglutaminase, gamma-glutamyl transpeptidase, phosphorylase α, phosphorylase β, dextransucrase, levansucrase, sucrose phosphorylase, glycogen synthase, UDP-glucuronyltransferase, galactosyl transferase, nucleoside phosphorylase, α- and β-amylase, amyloglucosidase, cellulase, dextranase, chitinase, pectinase, lysozyme, neuraminidase, xylanase, α- and β-glucosidase, α- and β-galactosidase, α- and β-mannosidase, invertase, trehalase, β-N-acetylglucosaminidase, β-glucuronidase, hyaluronidase, β-xylosidase, hesperidinase, pullulanase, α-fucosidase, agarase, endoglycosidase F, NADase, glycopeptidase F, thioglucosidase, catechol A-methyltransferase, aspartate transcarbamylase, omithine transcarbamylase, S-adenosylhomocysteine hydrolase.

16. A method according to claim 4 comprising:
(a) conducting a series of biocatalytic reactions by mixing biocatalysts with a starting compound to produce a reaction mixture and thereafter a library of modified starting compounds;
(b) testing the library of modified starting compounds to determine if a modified starting compound is present within the library which exhibits a desired activity;
(c) identifying the specific biocatalytic reactions which produce the modified starting compound of desired activity by systematically eliminating each of the biocatalytic reactions used to produce a portion of the library, and then testing the compounds produced in the portion of the library for the presence or absence of the modified starting compound with the desired activity; and
(d) repeating the specific biocatalytic reactions which produce the modified compound of desired activity and determining the chemical composition of the reaction product.

17. A method according to claim 16, wherein
(a) the biocatalytic reactions are conducted with a group of biocatalysts that react with distinct structural moieties found within the structure of a starting compound,
(b) each biocatalyst is specific for one structural moiety or a group of related structural moieties; and
(c) each biocatalyst reacts with many different starting compounds which contain the distinct structural moiety.

18. A method according to claim 1 comprising:
(a) conducting a series of biocatalytic reactions on a starting compound to produce a library of modified compounds;
(b) providing a high affinity receptor for a starting compound of desired activity and exposing the modified compounds formed after each biocatalytic reaction to the high affinity receptor;
(c) isolating the high affinity receptor-modified starting compound complex from the biocatalytic reaction mixture;
(d) dissociating the complex into its component parts; and
(e) determining the chemical composition of the dissociated modified starting compound.

19. A method according to claim 1 comprising:
(a) adding a high affinity receptor to a starting compound to form a biocatalytic reaction mixture at a concentration that allows for essentially all of the high affinity receptor to bind with the starting compound and about an equal molar amount of starting compound remaining free in solution and available for biocatalytic modification;
(b) producing a modified starting compound exhibiting a higher binding affinity than the starting compound resulting in the displacement of the starting compound from the high affinity receptor;
(c) forming a complex of the modified starting compound and the high affinity receptor, thereby protecting the modified starting compound from further biocatalytic reaction;
(d) isolating the complex from the biocatalytic reaction mixture;
(e) dissociating the complex into its component parts;
(f) determining the chemical composition of the dissociated modified starting compound; and
(g) repeating steps (a) through (b) to produce a library of modified starting compounds.

20. A method according to claim 19, wherein the high affinity receptor is present on the surface or contained within a living cell or immobilized to a natural or artificial support.

21. A method according to claim 1, wherein the biocatalytic reactions are used in combination with non-specific chemical reactions to produce a library of modified starting compounds.

22. A method according to claim 1, further comprising:
(a) exposing the reaction mixture to a drug screening device that measures the binding of compounds with desired activity to localized or immobilized receptor molecules or cells;
(b) correlating a positive measurement from the drug screening device with the sequence of biocatalytic reactions used to synthesize the reaction mixture and the specific reaction sequence producing the modified starting compound with desired activity; and
(c) repeating the biocatalytic reaction sequence to produce the modified starting compound of desired activity and to determine its chemical composition.

23. A method according to claim 12, wherein the biocatalytic reaction is performed with a biocatalyst immobilized to magnetic particles forming a magnetic biocatalyst, the method further comprising:
(a) initiating the biocatalytic reaction by combining the immobilized biocatalyst with substrate(s), cofactor(s) and solvent/buffer conditions used for a specific biocatalytic reaction;
(b) removing the magnetic biocatalyst from the biocatalytic reaction mixture to terminate the biocatalytic reaction, which is accomplished by applying an external magnetic field causing the magnetic particles with the immobilized biocatalyst to be attracted to and concentrate at the source of the magnetic field, thus effectively separating the magnetic biocatalyst from the bulk of the biocatalytic reaction mixture and allowing for the transferal of the reaction mixture minus the magnetic biocatalyst from a first reaction vessel to a second reaction vessel, leaving the magnetic biocatalyst in the first reaction vessel;
(c) conducting a second biocatalytic reaction, completely independent of the first biocatalytic reaction, by combining a second biocatalyst immobilized to magnetic particles with the second reaction vessel containing the biocatalytic reaction mixture transferred from the first reaction vessel; and
(d) repeating steps a) through c) to accomplish a sequential series of distinct and independent biocatalytic reactions and produce a corresponding series of modified starting compounds.

24. A method according to claim 23, wherein the biocatalytic reaction is performed with a biocatalyst immobilized to a particle and the biocatalyst is removed from the biocatalytic reaction mixture by centrifugation or filtration.

25. A method according to claim 12 using an automated robotic device, the automated robotic device comprised of:
(a) an XY table with an attached XYZ pipetting boom to add volumetric amounts of enzyme, substrate, cofactor, solvent solutions and assay reagents from reagent vessels positioned on the XY table to reaction and assay vessels positioned on the same XY table;
(b) an XYX reaction-vessel transfer boom attached to the same XY table used to transfer reaction and assay vessels positioned on the XY table to different locations on the XY table;
(c) a temperature incubation block attached to the same XY table to house the reaction and assay vessels during reaction incubations and control the temperature of the reaction mixtures;
(d) a magnetic separation block attached to the same XY table to separate the biocatalyst immobilized to magnetic particles from the biocatalytic mixture by applying an external magnetic field causing the magnetic particles to be attracted to and concentrate at the source of the magnetic field, thus effectively separating them from the bulk of the biocatalytic reaction mixture; and
(e) a programmable microprocessor interfaced to the XYZ pipetting boom, and XYZ reaction vessel transfer boom, the temperature block and the magnetic separation block to precisely control and regulate all movements and operations of these functional units in performing biocatalytic reactions to produce modified starting compounds and assays to determine desired activities.

26. A method according to claim 1 comprising:
(a) mixing a starting drug compound with a high affinity receptor at approximately one-half the molar concentration of the starting drug compound to allow essentially all of the high affinity receptor to bind to the starting drug compound and leave an equal molar amount of starting drug compound free in solution and available for biocatalytic modification;
(b) producing a modified drug compound from the biocatalytic reaction between the starting drug compound and the receptor, the modified drug compound having a higher binding affinity than the starting drug compound;
(c) displacing the starting drug compound from the receptor with the modified drug compound;
(d) protecting the bound modified drug compound from further biocatalytic reactions;
(e) dissociating the complex of the modified drug compound and receptor into its component parts;
(f) determining the chemical composition of the dissociated modified starting drug compound; and
(g) repeating steps a) through e) and thus conduct a series of biocatalytic reactions on a starting drug compound to produce a library of modified drug compounds with higher binding affinities.

27. A method according to claim 26, wherein the high affinity receptor is present on the surface or contained within a living cell or immobilized to a natural or artificial support.

28. A method according to claim 4, wherein the testing of the library of modified starting compounds is selected from a group of assays consisting of those listed in Table VI.

29. A method according to claim 12, wherein the biocatalytic reaction is performed with a biocatalyst immobilized to magnetic particles forming a magnetic biocatalyst whereas:
(a) the biocatalytic reaction is initiated by combining the immobilized biocatalyst with the following to form the biocatalytic reaction mixture: substrate(s), cofactor(s) and solvent/buffer conditions used for the specific biocatalytic reaction;
(b) the biocatalytic reaction is terminated by removing the magnetic biocatalyst from the biocatalytic reaction mixture, which is accomplished by applying an external magnetic field causing the magnetic particles with the immobilized biocatalyst to be attracted to and concentrate at the source of the magnetic field, thus effectively separating the magnetic biocatalyst from the bulk of the biocatalytic reaction mixture and allowing for the removal of the reaction mixture minus the magnetic biocatalyst to a second reaction vessel, leaving the magnetic biocatalyst in the first reaction vessel;
(c) a second biocatalytic reaction, completely independent of the first biocatalytic reaction, is initiated by adding a second biocatalyst immobilized to magnetic particles to the second reaction vessel containing the biocatalytic reaction mixture from the first biocatalytic reaction minus the magnetic biocatalyst used in the first biocatalytic reaction;
(d) the above described biocatalytic reaction cycle is repeated thus accomplishing a series of distinct and independent biocatalytic reactions producing a corresponding series of modified drug compounds;
(e) a magnetic separation block attached to the same XY table as above to separate the biocatalyst immobilized to magnetic particles from the biocatalytic reaction mixture by applying an external magnetic field causing the magnetic particles to be attracted to and concentrate at the source of the magnetic field, thus effectively separating them from the bulk of the biocatalytic reaction mixture; and
(f) a programmable microprocessor interfaced to the XYZ pipetting boom, the XYZ reaction vessel transfer boom, the temperature block and the magnetic separation block to precisely control and regulate all movements and operations of these functional units in performing biocatalytic reactions to produce modified starting compounds and assays to determine desired activities.

30. A method according to any of claims 1 to 29, wherein the starting compounds are soluble in the reaction mixture.

31. A method according to any one of claims 1 to 29, wherein the starting compounds and resulting derivatives are soluble, and remain soluble for use in screening procedures.

## Patentansprüche

1. Verfahren zum Erzeugen einer Bibliothek von nicht-biologischen organischen Verbindungen mit einer gewünschten Aktivität, umfassend:
(a) Auswählen einer Ausgangsverbindung, die eine Wirkstoffaktivität aufweist oder von der man annimmt, dass sie eine Wirkstoffaktivität für eine bestimmte Krankheit oder Störung aufweist;
(b) Analyse der Ausgangsverbindung hinsichtlich ihres Gehalts an funktionellen Gruppen;
(c) Hinzufügen von mindestens einem Biokatalysator, von dem bekannt ist, dass er auf mindestens eine ausgewählte funktionelle Gruppe der Verbindung einwirkt;
(d) Auswählen von Reaktionsbedingungen, unter denen mindestens ein Biokatalysator mit der ausgewählten funktionellen Gruppe der Verbindung reagiert, um mindestens eine modifizierte Verbindung zu erhalten;
(e) Hinzufügen von mindestens einem zweiten Biokatalysator zur modifizierten Verbindung, von dem bekannt ist, dass er auf mindestens eine ausgewählte funktionelle Gruppe der Verbindung einwirkt, um eine zweite Population von modifizierten Verbindungen zu erzeugen; und
(f) Wiederholen des Verfahrens, um eine Bibliothek von modifizierten Verbindungen zu erhalten,
unter der Bedingung, dass es sich bei der Ausgangsverbindung nicht um ein Peptid oder ein Protein handelt, sofern der Biokatalysator eine Protease ist.

2. Verfahren nach Anspruch 1, wobei der zweite Biokatalysator zu der modifizierten Verbindung getrennt von dem Biokatalysator aus Schritt (d) hinzugefügt wird.

3. Verfahren nach Anspruch 1, wobei der zusätzliche Biokatalysator zusammen mit dem Biokatalysator aus Schritt (d) zur Ausgangsverbindung hinzugefügt wird.

4. Verfahren nach Anspruch 1, weiter umfassend:
(g) Screenen der modifizierten Verbindungen in der Bibliothek bezüglich dem Vorhandensein einer Aktivität.

5. Verfahren nach Anspruch 1, wobei die in der Ausgangsverbindung enthaltene funktionelle Gruppe aus der Gruppe bestehend aus Hydroxylgruppen, Aldehydgruppen, Ketongruppen, Aminogruppen, Carboxylgruppen, Thiolgruppen, aromatischen Gruppen, Kohlenhydratgruppen, Estergruppen, Peptidgruppen, Sulfatgruppen, Phosphatgruppen, Halogengruppen, aromatischen Amingruppen und Phenolgruppen ausgewählt wird.

6. Verfahren nach Anspruch 1, wobei die Biokatalysatoren aus der Gruppe bestehend aus Dehydrogenasen, Dehydratasen, Oxydasen, Esterasen, Lipasen, Proteasen, Sulfatasen, Phosphatasen, Acylasen, Laktamasen, Nukleasen, Acyl-Transferasen, Transglycosylasen primärer und sekundärer Alkohole, Etherificasen primärer und sekundärer Alkohole, Acylasen primärer und sekundärer Amine und Esterificasen ausgewählt werden.

7. Verfahren nach Anspruch 1, wobei das Verfahren automatisiert ist.

8. Verfahren nach Anspruch 4, wobei das Screening automatisiert ist.

9. Verfahren nach Anspruch 1, wobei sich die Biokatalysatoren in einer Formulierung befinden, die aus der Gruppe bestehend aus Rohenzymen, gereinigten Enzymen, zellulären Lysate-Präparaten, teilweise gereinigten Lysate-Präparaten, lebenden Zellen und intakten nichtlebenden Zellen ausgewählt wird.

10. Verfahren nach Anspruch 1, wobei die Reaktion zwischen den Verbindungen und den Biokatalysatoren in einem Medium stattfindet, das aus der Gruppe bestehend aus Lösungs-, Suspensions- und Immobilisierungsmitteln ausgewählt wird.

11. Verfahren nach Anspruch 10, wobei der Biokatalysator immobilisiert ist.

12. Verfahren nach Anspruch 11, wobei der Biokatalysator an einem magnetischen Partikel immobilisiert ist.

13. Verfahren nach Anspruch 1, weiterhin umfassend die Reaktion der Verbindung unter Verwendung von unspezifischen chemischen Reaktionen, die aus der Gruppe bestehend aus Hydroxylierung, Oxidation, Reduktion, Dehydratation, Hydratation, Hydrolyse, Veresterung, Umesterung, Aldolkondensation, reduktive Aminierung, Ammonolyse, Dehydrohalogenierung, Halogenierung, Acylierung, Acyl-Substitution, aromatischer Substitution, Grignard-Synthese und Friedel-Crafis-Acylierung ausgewählt werden.

14. Verfahren nach Anspruch 1 weiterhin umfassend, dass zu der Reaktion der Verbindung mit mindestens einem Biokatalysator mindestens ein Cofaktor für einen Biokatalysator hinzugefügt wird.

15. Verfahren nach Anspruch 1, wobei die Biokatalysatoren aus der Gruppe bestehend aus Alkoholdehydrogenasen, Glyceroldehydrogenasen, Glycerol-3-phosphatdehydrogenasen, Xylulosereduktasen, Polyol-Dehydrogenasen, Sorbitol-Dehydrogenasen, Glyoxylat-Reduktasen, Lactatdehydrogenasen, Glyceratdehydrogenasen, β-Hydroxybutyrat-Dehydrogenasen, Malatdehydrogenasen, Glukosedehydrogenasen, Glukose-6-phosphat-Dehydrogenasen, 3α- und 3β-Hydroxysteroid-Dehydrogenasen, 3α-, 20β-Hydroxysteroid-Dehydrogenasen, Fucosedehydrogenasen, Cytochrom-abhängige Lactatdehydrogenasen, Galactoseoxidasen, Glucoseoxidasen, Cholesteroloxidasen, Alkohol-Oxidasen, Glycolat-Oxidasen, Xanthin-Oxidasen, Fructose-Dehydrogenasen, Phospholipase A, Acetylesterasen, Acetylcholinesterasen, Butyryl-Cholinesterasen, Pektinesterasen, Cholesterolesterasen, Glyoxalasen II, alkalischen Phosphatasen, sauren Phosphatasen, Glucose-6-Phosphatasen, Fructose-1,6-diphosphatasen, Ribonukleasen, Deoxyribonucleasen, Chondro-4-Sulfatasen, Chondro-6-Sulfatasen, Leuzinaminopeptidasen, Carboxypeptidasen A, Carboxypeptidasen B, Carboxypeptidasen Y, Carboxypeptidasen W, Prolidasen, Kathepsin C, Chymotrypsin, Trypsin, Elastasen, Subtilisin, Papain, Pepsin, Ficin, Bromelin, Rennin, Proteinase A, Kollagenasen, Urokinasen, Asparaginasen, Glutaminasen, Ureasen, Acylasen I, Pencillinasen, Cephalosporinasen, Creatininasen, Guanasen, Adenosine-Deaminasen, Kreaminen, Deaminasen, anorganischen Pyrophosphatasen, ATPasen, Cholinacetyltransferasen, Carnitin, Acetyltransferasen, Phosphotransacetylasen, Chloramphenicol-Acetyltransferasen, Transglutaminasen, Gamma-Glutamyltranspeptidasen, α-Phosphorylase, β-Phosphorylase, Dextransucrasen, Lävansucrasen, Saccharosephosphorylasen, Glycogensynthasen, UDP-Glucuronyltransferasen, Galactosyltransferasen, Nukleosidphosporylasen, α- und β-Amylasen, Amyloglucosidasen, Cellulasen, Dextranasen, Chitinasen, Pectinasen, Lysozymen, Neuraminidasen, Xylanasen, α- und β-Glucosidasen, α- und β-Galactosidasen, α- und β-Mannosidasen, Invertasen, Trehalasen, β-N-Acetylglucosaminidasen, β-Glucuronidasen, Hyaluronidasen, β-Xylosidasen, Hesperinidasen, Pullulanasen, α-Fucosidasen, Agarasen, Endoglycosidasen F, NADasen, Glycopeptidasen F, Thioglucosidasen, Catechol A-Methyltransferasen, Aspartattranskarbamylasen, Ornithin-Transcarbamylasen, S-Adenosylhomocystein Hydrolasen ausgewählt werden.

16. Verfahren nach Anspruch 4, Folgendes umfassend:
(a) Durchführen einer Reihe von biokatalytischen Reaktionen durch Mischung von Biokatalysatoren mit einer Ausgangsverbindung, um ein Reaktionsgemisch und anschließend eine Bibliothek von modifizierten Ausgangsverbindungen zu erzeugen;
(b) Untersuchen der Bibliothek von modifizierten Ausgangsverbindungen um nachzuweisen, ob eine modifizierte Ausgangsverbindung innerhalb der Bibliothek vorhanden ist, die eine gewünschte Aktivität aufweist;
(c) Identifizieren der spezifischen biokatalytischen Reaktionen, welche die modifizierte Ausgangsverbindung mit der gewünschten Aktivität erzeugen durch systematisches Entfernen der biokatalytischen Reaktionen, die zur Erstellung eines Teils der Bibliothek eingesetzt werden und Testen der in dem Teil der Bibliothek hergestellten Verbindungen auf das Vorhandensein oder Nichtvorhandensein der modifizierten Ausgangsverbindung mit der gewünschten Aktivität; und
(d) Wiederholen der spezifischen biokatalytischen Reaktionen, welche die modifizierte Verbindung mit der gewünschten Aktivität erzeugen, und Bestimmung der chemischen Zusammensetzung des Reaktionsprodukts.

17. Verfahren nach Anspruch 16, wobei
(a) die biokatalytischen Reaktionen mit einer Gruppe von Biokatalysatoren durchgeführt werden, die ihrerseits mit unterschiedlichen strukturellen Anteilen reagieren, welche innerhalb der Struktur einer Ausgangsverbindung nachgewiesen wurden,
(b) jeder Biokatalysator für einen strukturellen Anteil oder für eine Gruppe von verwandten strukturellen Anteilen spezifisch ist; und
(c) jeder Biokatalysator mit vielen unterschiedlichen Ausgangsverbindungen reagiert, die den unterschiedlichen strukturellen Anteil enthalten.

18. Verfahren nach Anspruch 1, Folgendes umfassend:
(a) Durchführen einer Reihe von biokatalytischen Reaktionen einer Ausgangsverbindung, um eine Bibliothek von modifizierten Verbindungen zu erzeugen;
(b) Bereitstellen eines hochaffinen Rezeptors für eine Ausgangsverbindung mit einer gewünschten Aktivität und Exposition der modifizierten Verbindungen, die nach jeder biokatalytischen Reaktion gebildet werden, an den hochaffinen Rezeptor;
(c) Isolieren des durch den hochaffinen Rezeptor modifizierten Komplexes der Ausgangsverbindung von dem biokatalytischen Reaktionsgemisch;
(d) Dissoziieren des Komplexes in dessen Bestandteile; und
(e) Bestimmen der chemischen Zusammensetzung der dissoziierten modifizierten Ausgangsverbindung;

19. Verfahren nach Anspruch 1, Folgendes umfassend:
(a) Hinzufügen eines hochaffinen Rezeptors zu einer Ausgangsverbindung, um ein biokatalytisches Reaktionsgemisch mit einer Konzentration zu bilden, bei der sich im Wesentlichen der gesamte hochaffine Rezeptor mit der Ausgangsverbindung verbindet, wobei etwa ein gleich großer molarer Anteil der Ausgangsverbindung in der Lösung ungebunden bleibt und für die biokatalytische Modifikation zur Verfügung steht;
(b) Erzeugen einer modifizierten Ausgangsverbindung, die eine höhere Bindungsaffinität aufweist als die Ausgangsverbindung mit der Folge, dass die Ausgangsverbindung aus dem hochaffinen Rezeptor verdrängt wird;
(c) Bilden eines Komplexes aus der modifizierten Ausgangsverbindung und dem hochaffinen Rezeptor, wodurch die modifizierte Ausgangsverbindung vor einer weiteren biokatalytischen Reaktion geschützt wird;
(d) Isolieren des Komplexes aus dem biokatalytischen Reaktionsgemisch;
(e) Dissoziieren des Komplexes in dessen Bestandteile;
(f) Bestimmen der chemischen Zusammensetzung der dissoziierten modifizierten Ausgangsverbindung; und
(g) Wiederholen der Schritte (a) bis (b) zum Zwecke der Erzeugung einer Bibliothek von modifizierten Ausgangsverbindungen.

20. Verfahren nach Anspruch 19, wobei der hochaffine Rezeptor an der Oberfläche einer lebenden Zelle ausgebildet ist oder sich in einer lebenden Zelle befindet oder fest an einen natürlichen oder künstlichen Träger gebunden ist.

21. Verfahren nach Anspruch 1, wobei der Einsatz der biokatalytischen Reaktionen in Verbindung mit unspezifischen chemischen Reaktionen erfolgt, um eine Bibliothek von modifizierten Ausgangsverbindungen zu erzeugen.

22. Verfahren nach Anspruch 1, weiterhin umfassend:
(a) Untersuchen des Reaktionsgemisches mittels eines Wirkstofftestgeräts, das die Bindungsfähigkeit von Verbindungen mit einer gewünschten Aktivität gegenüber lokalisierten oder fest gebundenen Rezeptormolekülen oder -zellen misst;
(b) Korrelieren einer positiven Messung des Wirkstofftestgeräts mit der zur Synthese des Reaktionsgemisches verwendeten Sequenz von biokatalytischen Reaktionen, und der spezifischen Reaktionssequenz zur Herstellung der modifizierten Ausgangsverbindung mit einer gewünschten Aktivität; und
(c) Wiederholen der Sequenz von biokatalytischen Reaktionen zur Erzeugung der modifizierten Ausgangsverbindung mit einer gewünschten Aktivität und zur Bestimmung ihrer chemischen Zusammensetzung.

23. Verfahren nach Anspruch 12, wobei die biokatalytische Reaktion mit einem Biokatalysator ausgeführt wird, der durch die Immobilisation an magnetische Partikel einen magnetischen Biokatalysator bildet, und das Verfahren weiterhin umfasst:
(a) Initiieren der biokatalytischen Reaktion durch Kombination des immobilisierten Biokatalysators mit Substrat(en), Cofaktor(en) und Lösungsmittel-/Pufferbedingungen, die für eine spezifische biokatalytische Reaktion eingesetzt werden;
(b) Entfernen des magnetischen Biokatalysators aus dem biokatalytischen Reaktionsgemisch, um die biokatalytische Reaktion zu beenden, welches durch den Einsatz eines externen magnetischen Feldes erreicht wird, wobei die magnetischen Partikel mit dem immobilisierten Biokatalysator von der Quelle des magnetischen Feldes angezogen und dort konzentriert werden, wodurch erreicht wird, dass der magnetische Biokatalysator wirksam von der Hauptmenge des biokatalytischen Reaktionsgemisches getrennt wird und es somit möglich ist, das Reaktionsgemisch abzüglich des magnetischen Biokatalysators von einem ersten Reaktionsgefäß zu einem zweiten Reaktionsgefäß zu übertragen, wobei der magnetische Biokatalysator in dem ersten Reaktionsgefäß verbleibt;
(c) Durchführen einer zweiten biokatalytischen Reaktion, die vollkommen unabhängig von der ersten biokatalytischen Reaktion ist, durch Einbringen eines zweiten fest an magnetische Partikel gebundenen Biokatalysators in das zweite Reaktionsgefäß, welches das aus dem ersten Reaktionsgefäß übertragene biokatalytische Reaktionsgemisch enthält; und
(d) Wiederholen der Schritte a) bis c), um eine sequentielle Reihe von unterschiedlichen und unabhängigen biokatalytischen Reaktionen auszuführen und eine entsprechende Reihe von modifizierten Ausgangsverbindungen zu erzeugen.

24. Verfahren nach Anspruch 23, wobei die biokatalytische Reaktion mit einem Biokatalysator ausgeführt wird, der fest an ein Partikel gebunden ist und der Biokatalysator aus dem biokatalytischen Reaktionsgemisch durch Zentrifugierung oder Filtration entfernt wird.

25. Verfahren nach Anspruch 12 unter Verwendung eines automatisierten Robotermechanismus, wobei der automatisierte Robotermechanismus Folgendes umfasst:
(a) einen XY-Tisch mit einem angeschlossenen XYZ-Pipettierarm, um volumetrische Mengen von Enzymen, Substraten, Cofaktoren, Lösungsmittellösungen und Versuchsreagenzien aus den auf dem XY-Tisch positionierten Reagenzgefäßen zu Reaktions- und Versuchsgefäßen hinzuzufügen, die auf demselben XY-Tisch positioniert sind;
(b) ein XYZ-Übertragungsarm für Reaktionsgefäße, der an demselben XY-Tisch angeordnet ist und dazu dient, Reaktions- und Versuchsgefäße auf dem XY-Tisch an andere Positionen auf dem XY-Tisch zu transportieren;
(c) ein temperierbarer Inkubationsblock, der an demselben XY-Tisch angeordnet ist, um die Reaktions- und Versuchsgefäße während der Inkubation der Reaktionen aufzunehmen und die Temperatur der Reaktionsgemische zu steuern;
(d) ein Magnetscheider, der an demselben XY-Tisch angeordnet ist, um den fest an die magnetischen Partikel gebundenen Biokatalysator aus dem biokatalytischen Gemisch durch den Einsatz eines externen magnetischen Feldes abzuscheiden, wobei die magnetischen Partikel von der Quelle des magnetischen Feldes angezogen und dort angesammelt werden, und somit von der Hauptmenge des biokatalytischen Reaktionsgemisches wirksam abgesondert werden; und
(e) ein programmierbarer Mikroprozessor, der mit dem XYZ-Pipettierarm, dem temperierbaren Inkubationsblock und dem Magnetabscheider verbunden ist, um alle Bewegungen und Vorgänge dieser Funktionseinheiten bei der Durchführung von biokatalytischen Reaktionen zur Erzeugung modifizierter Ausgangsverbindungen und von Versuchen, die zur Bestimmung gewünschter Aktivitäten dienen, genau zu steuern und zu regulieren.

26. Verfahren nach Anspruch 1, umfassend:
(a) Mischen einer Ausgangswirkstoffverbindung mit einem hochaffinen Rezeptor, der etwa die halbe Molkonzentration der Ausgangswirkstoffverbindung aufweist, um im Wesentlichen die Bindung des gesamten hochaffinen Rezeptors an die Ausgangswirkstoffverbindung zu ermöglichen, wobei ein gleich großer molarer Anteil der Ausgangswirkstoffverbindung in der Lösung ungebunden bleibt und für biokatalytische Modifikationen zur Verfügung steht;
(b) Erzeugen einer modifizierten Wirkstoffverbindung aus der biokatalytischen Reaktion zwischen der Ausgangswirkstoffverbindung und dem Rezeptor, wobei die modifizierte Wirkstoffverbindung eine höhere Bindungsaffinität aufweist als die Ausgangswirkstoffverbindung;
(c) Verdrängen der Ausgangswirkstoffverbindung von dem Rezeptor mit der modifizierten Wirkstoffverbindung;
(d) Schützen der gebundenen modifizierten Wirkstoffverbindung vor weiteren biokatalytischen Reaktionen;
(e) Dissoziieren des Komplexes aus modifizierter Wirkstoffverbindung und Rezeptor in dessen Bestandteile;
(f) Bestimmen der chemischen Zusammensetzung der dissoziierten modifizierten Ausgangswirkstoffverbindung; und
(g) Wiederholung der Schritte a) bis e) und somit Durchführung einer Reihe von biokatalytischen Reaktionen mit einer Ausgangswirkstoffverbindung, um eine Bibliothek von modifizierten Ausgangswirkstoffverbindungen mit höheren Bindungsaffinitäten zu erzeugen.

27. Verfahren nach Anspruch 26, wobei der hochaffine Rezeptor an der Oberfläche einer lebenden Zelle ausgebildet ist oder sich in einer lebenden Zelle befindet oder fest an einen natürlichen oder künstlichen Träger gebunden ist.

28. Verfahren nach Anspruch 4, wobei das Testen der Bibliothek modifizierter Ausgangsverbindungen aus der Gruppe von Versuchen ausgewählt wird, die sich aus den Versuchen zusammensetzt, die in Tabelle VI aufgeführt sind.

29. Verfahren nach Anspruch 12, wobei die biokatalytische Reaktion mit einem Biokatalysator durchgeführt wird, der durch die feste Bindung an magnetische Partikel einen magnetischen Biokatalysator bildet, und wobei:
(a) die biokatalytische Reaktion durch Kombination des fest gebundenen Biokatalysators mit Folgenden initiiert wird, um das biokatalytische Reaktionsgemisch zu bilden: Substrate(n), Cofaktor(en) und Lösungs-/Pufferbedingungen, die für die spezifische biokatalytische Reaktion eingesetzt werden;
(b) die biokatalytische Reaktion durch Entfernen des magnetischen Biokatalysators aus dem biokatalytischen Reaktionsgemisch beendet wird, welches durch den Einsatz eines externen magnetischen Feldes erzielt wird, wobei die magnetischen Partikel mit dem fest gebundenen Biokatalysator von der Quelle des magnetischen Feldes angezogen und dort konzentriert werden, wodurch erreicht wird, dass der magnetische Biokatalysator wirksam von der Hauptmenge des biokatalytischen Reaktionsgemisches getrennt wird und es somit möglich ist, das Reaktionsgemisch abzüglich des magnetischen Biokatalysators von einem ersten Reaktionsgefäß zu einem zweiten Reaktionsgefäß zu übertragen, wobei der magnetische Biokatalysator in dem ersten Reaktionsgefäß verbleibt;
(c) eine zweite biokatalytische Reaktion initiiert wird, die vollkommen unabhängig von der ersten biokatalytischen Reaktion ist, indem ein zweiter fest an magnetische Teilchen gebundener Biokatalysator zu dem zweiten Reaktionsgefäß hinzugefügt wird, in dem das biokatalytische Reaktionsgemisch aus der ersten biokatalytischen Reaktion abzüglich des in der ersten biokatalytischen Reaktion eingesetzten magnetischen Biokatalysators enthalten ist;
(d) der oben beschriebene biokatalytische Reaktionszyklus wiederholt wird, und somit eine Reihe von unterschiedlichen und unabhängigen biokatalytischen Reaktionen zur Herstellung einer entsprechende Reihe von modifizierten Wirkstoffverbindungen ausgeführt wird;
(e) ein Magnetscheider, der wie oben dargestellt an demselben XY-Tisch angeordnet ist, zur Abtrennung des an magnetische Partikel fest gebundenen Biokatalysators von dem biokatalytischen Reaktionsgemisch durch Einsatz eines externen magnetisches Feldes, wobei die magnetischen Partikel von der Quelle des magnetischen Feldes angezogen und dort angesammelt werden und die magnetischen Partikel von der Hauptmenge des biokatalytischen Reaktionsgemisches folglich wirksam getrennt werden; und
(f) ein programmierbarer Mikroprozessor, der mit dem XYZ-Pipettierarm, dem Übertragungsarm für XYZ-Reaktionsgefäße, dem Temperaturblock und dem Magnetabscheider verbunden ist, um alle Bewegungen und Vorgänge dieser Funktionseinheiten bei der Durchführung von biokatalytischen Reaktionen zur Erzeugung modifizierter Ausgangsverbindungen und Versuchen, die zur Bestimmung gewünschter Aktivitäten dienen, genau zu steuern und zu regulieren.

30. Verfahren nach einem der Ansprüche 1 bis 29, wobei die Ausgangsverbindungen in dem Reaktionsgemisch löslich sind.

31. Verfahren nach einem der Ansprüche 1 bis 29, wobei die Ausgangsverbindungen und die sich ergebenden Derivate löslich sind und für den Einsatz in Screening-Verfahren löslich bleiben.

## Revendications

1. Procédé pour générer une banque de composés organiques non-biologiques dotés d'une activité souhaitée, comprenant :
(a) la sélection d'un composé de départ qui présente une activité en tant que médicament, ou qui est supposé présenter une activité en tant que médicament pour une maladie ou un trouble donné ;
(b) l'analyse du composé de départ du point de vue de son contenu en groupes fonctionnels ;
(c) l'addition au composé de départ, d'au moins un biocatalyseur qui est connu pour agir sur au moins un groupe fonctionnel sélectionné dudit composé ;
(d) le choix des conditions réactionnelles dans lesquelles ledit «au moins un biocatalyseur» réagira avec le groupe fonctionnel sélectionné sur le composé, pour donner au moins un composé modifié ;
(e) l'addition au composé modifié, d'au moins un second catalyseur qui est connu pour agir sur au moins un groupe fonctionnel choisi sur le composé, pour produire une seconde population de composés modifiés ; et
(f) la répétition du procédé pour obtenir une banque de composés modifiés,
à la condition que le composé de départ ne soit pas un peptide ou une protéine lorsque le biocatalyseur est une protéase.

2. Procédé selon la revendication 1, dans lequel le second biocatalyseur est additionné au composé modifié, séparément du biocatalyseur de l'étape (d).

3. Procédé selon la revendication 1, dans lequel le biocatalyseur est additionné au composé de départ, avec le biocatalyseur de l'étape (d).

4. Procédé selon la revendication 1, comprenant aussi
(g) le criblage des composés modifiés de la banque, du point de vue d'une activité.

5. Procédé selon la revendication 1, dans lequel le groupe fonctionnel sur le composé de départ est choisi au sein de l'ensemble comprenant les groupes hydroxyle, les groupes aldéhyde, les groupes cétone, les groupes amino, les groupes carboxyle, les groupes thiol, les groupes aromatiques, les groupes carbohydrate, les groupes ester, les groupes peptide, les groupes sulfate, les groupes phosphate, les groupes halogène, les groupes amine aromatique et les groupes phénol.

6. Procédé selon la revendication 1, dans lequel les biocatalyseurs sont choisis au sein du groupe comprenant les déshydrogénases, les déshydratases, les oxydases, les estérases, les lipases, les protéases, les sulfatases, les phosphatases, les acylases, les lactamases, les nucléases, les acyl-transférases, les transglycosylases d'alcools primaires et secondaires, les éthérificases d'alcools primaires et secondaires, les acylases d'amines primaires et secondaires et les estérificases.

7. Procédé selon la revendication 1, qui est automatisé.

8. Procédé selon la revendication 4, dans lequel le criblage est automatisé.

9. Procédé selon la revendication 1, dans lequel les biocatalyseurs sont dans une formulation choisie au sein du groupe comprenant les enzymes brutes, les enzymes purifiées, les préparations de lysats cellulaires, les préparations de lysats cellulaires partiellement purifiées, les cellules vivantes et les cellules non-vivantes intactes.

10. Procédé selon la revendication 1, dans lequel les composés sont mis en réaction avec les biocatalyseurs dans un milieu choisi au sein du groupe comprenant les solutions, les suspensions et les moyens d'immobilisation.

11. Procédé selon la revendication 10, dans lequel le biocatalyseur est immobilisé.

12. Procédé selon la revendication 11, dans lequel le biocatalyseur est immobilisé sur une particule magnétique.

13. Procédé selon la revendication 1, comprenant aussi la réaction du composé par mise en oeuvre de réactions chimiques non-spécifiques choisies au sein du groupe comprenant l'hydroxylation, l'oxydation, la réduction, la déshydratation, l'hydratation, l'hydrolyse, l'estérification, la trans-estérification, la condensation aldolique, l'amination réductrice, l'ammonolyse, la déshydrohalogénation, l'halogénation, l'acylation, la substitution acylique, la substitution aromatique, la synthèse de Grignard et l'acylation de Friedel et Crafts.

14. Procédé selon la revendication 1, comprenant aussi l'addition, lors de la réaction entre le composé et au moins un biocatalyseur, d'au moins un co-facteur pour un biocatalyseur.

15. Procédé selon la revendication 1, dans lequel les biocatalyseurs sont choisis au sein du groupe comprenant l'alcool déshydrogénase, la glycérol déshydrogénase, la glycérol-3-phosphate déshydrogénase, la xylulose réductase, la polyol déshydrogénase, la sorbitol déshydrogénase, la glyoxylate réductase, la lactate déshydrogénase, la glycérate déshydrogénase, la β-hydroxybutyrate déshydrogénase, la malate déshydrogénase, la glucose déshydrogénase, la glucose-6-phosphate déshydrogénase, la 3α- et la 3β-hydroxystéroïde déshydrogénase, la 3α-, 20β-hydroxystéroïde déshydrogénase, la fucose déshydrogénase, la lactate déshydrogénase cytochrome-dépendante, la galactose oxydase, la glucose oxydase, la cholestérol oxydase, l'alcool oxydase, la glycolate oxydase, la xanthine oxydase, la fructose déshydrogénase, le phospholipase A, l'acétylestérase, l'acétylcholinestérase, la butyrylcholinestérase, la pectinestérase, le cholestérolestérase, la glyoxalase II, la phosphatase alcaline, la phosphatase acide, la glucose-6-phosphatase, la fructose 1,6-diphosphatase, la ribonucléase, la désoxyribonucléase, la chondro-4-sulfatase, la chondro-6-sulfatase, la leucine aminopeptidase, la carboxypeptidase A, la carboxypeptidase B, la carboxypeptidase Y, la carboxypeptidase W, la prolidase, la cathepsine C, la chymotrypsine, la trypsine, l'élastase, la subtilisine, la papaïne, la pepsine, la ficine, la bromclaim, la rennine, la protéinase A, la collagénase, l'urokinase, l'asparaginase, la glutaminase, l'uréase, l'acylase I, la penicillinase, la céphalosporinase, la créatininase, la guanase, l'adénosine désaminase, la créamine désaminase, la pyrophosphatase inorganique, l'ATPase, la choline acétyltransférase, la camitine acétyltransférase, la phosphotransacétylase, la chloramphénicol acétyltransférase, la transglutaminase, la gamma-glutamyl transpeptidase, la phosphorylase α, la phosphorylase β, la dextransucrase, la levansucrase, la sucrose phosphorylase, la glycogène synthase, l'UDP-glucuronyltransférase, la galactosyl transférase, la nucléoside phosphorylase, l'α- et la β-amylase, l'amyloglucosidase, la cellulase, la dextranase, la chitinase, la pectinase, le lisozyme, la neuraminidase, la xylanase, l'α- et la β-glucosidase, l'α- et la β-galactosidase, l'α- et la β-mannosidase, l'invertase, la tréhalase, la β-N-acétylglucosaminidase, la β-glucuronidase, l'hyaluronidase, la β-xylosidase, l'hespérinidase, la pullulanase, l'α-fucosidase, l'agarase, l'endoglycosidase F, l'ADN-ase, la glycopeptidase F, la thioglucosidase, la catéchol A-méthyltransférase, l'aspartate transcarbamylase, l'omithine transcarbamylase, la S-adénosylhomocystéine hydrolase.

16. Procédé selon la revendication 4, comprenant :
(a) la mise en oeuvre d'une série de réactions biocatalytiques par le mélange de biocatalyseurs avec un composé de départ pour donner une mélange réactionnel, et après cela, une banque de composés de départ modifiés ;
(b) le contrôle de la banque de composés de départ modifiés pour déterminer si un composé de départ modifié est présent au sein de la banque et s'il présente l'activité désirée ;
(c) l'identification des réactions biocatalytiques spécifiques qui produisent le composé de départ modifié présentant l'activité désirée, par élimination systématique de chacune des réactions biocatalytiques utilisées pour produire une portion de la banque, et ensuite le contrôle des composés produits dans la portion de banque, du point de vue de la présence ou de l'absence du composé de départ modifié présentant l'activité désirée ; et
(d) la répétition des réactions biocatalytiques spécifiques qui produisent le composé modifié présentant l'activité désirée, et la détermination de la composition chimique du produit de la réaction.

17. Procédé selon la revendication 16, dans lequel
(a) les réactions biocatalytiques sont menées avec un groupe de biocatalyseurs qui réagissent avec des entités structurales distinctes se trouvant au sein de la structure d'un composé de départ,
(b) chaque biocatalyseur est spécifique d'une entité structurale ou d'un groupe d'entités structurales apparentées ; et
(c) chaque biocatalyseur réagit avec beaucoup de composés de départ différents qui contiennent l'entité structurale distincte.

18. Procédé selon la revendication 1, comprenant
(a) la mise en oeuvre d'une série de réactions biocatalytiques sur un composé de départ pour produire une banque de composés modifiés ;
(b) la mise en oeuvre d'un récepteur de haute affinité pour un composé de départ doté de l'activité désirée, et l'exposition des composés modifiés formés après chaque réaction biocatalytique, au récepteur de haute affinité ;
(c) l'isolement du complexe (récepteur de haute affinité)-(composé de départ modifié) à partir du mélange réactionnel biocatalytique ;
(d) la dissociation du complexe en ses parties composantes ; et
(e) la détermination de la composition chimique du composé de départ modifié, dissocié.

19. Procédé selon la revendication 1, comprenant :
(a) l'addition d'un récepteur de haute affinité à un composé de départ pour former un mélange réactionnel biocatalytique à une concentration qui permet à pratiquement la totalité du récepteur de haute affinité de se lier au composé de départ, et à environ une quantité molaire égale de composé de départ, de rester libre en solution et d'être disponible pour la modification biocatalytique.
(b) la production d'un composé de départ modifié présentant une affinité de liaison plus élevée que celle du composé de départ, ce qui entraîne le déplacement du composé de départ depuis le récepteur de haute affinité ;
(c) la formation d'un complexe du composé de départ modifié et du récepteur de haute affinité, ce qui protège le composé de départ modifié vis-à-vis d'une réaction biocatalytique ultérieure ;
(d) l'isolement du complexe à partir du mélange réactionnel biocatalytique ;
(e) la dissociation du complexe en ses parties composantes ;
(f) la détermination de la composition chimique du composé de départ modifié, dissocié ; et
(g) la répétition des étapes (a) à (b) pour produire une banque de composés de départ modifiés.

20. Procédé selon la revendication 19, dans lequel le récepteur à haute affinité est présent sur la surface, ou contenu au sein, d'une cellule vivante, ou bien est immobilisé sur un support naturel ou artificiel.

21. Procédé selon la revendication 1, dans lequel les réactions biocatalytiques sont utilisées en combinaison avec des réactions chimiques non-spécifiques pour produire une banque de composés de départ modifiés.

22. Procédé selon la revendication 1, comprenant aussi :
(a) l'exposition du mélange réactionnel à un dispositif de criblage de médicament qui mesure la liaison des composés dotés de l'activité désirée, avec des molécules récepteurs ou cellules localisés ou immobilisés ;
(b) la corrélation d'une mesure positive provenant du dispositif de criblage de médicament, avec la séquence des réactions biocatalytiques utilisées pour synthétiser le mélange réactionnel, et avec la séquence réactionnelle spécifique produisant le composé de départ modifié, doté de l'activité désirée ; et
(c) la répétition de la séquence de réactions biocatalytiques pour produire le composé de départ modifié, doté de l'activité désirée et pour déterminer sa composition chimique.

23. Procédé selon la revendication 12, dans lequel la réaction biocatalytique est effectuée avec un biocatalyseur immobilisé sur des particules magnétiques formant un biocatalyseur magnétique, ledit procédé comprenant également :
(a) l'initiation de la réaction biocatalytique en combinant le biocatalyseur immobilisé, avec un (ou des) substrat(s), un (ou des) cofacteur(s) et des conditions de solvant/tampon mis en oeuvre pour une réaction biocatalytique spécifique ;
(b) le retrait du biocatalyseur magnétique depuis le mélange réactionnel biocatalytique pour mettre fin à la réaction biocatalytique, ce qui est réalisé par l'application d'un champ magnétique externe qui provoque l'attraction des particules magnétiques avec le biocatalyseur immobilisé et leur concentration sur la source du champ magnétique, ce qui sépare efficacement le biocatalyseur magnétique de la masse du mélange réactionnel biocatalytique et qui permet le transfert du mélange réactionnel sans le biocatalyseur magnétique, depuis une première cuve réactionnelle vers une seconde cuve réactionnelle, en laissant le biocatalyseur magnétique dans la première cuve réactionnelle ; et
(c) la mise en oeuvre d'une seconde réaction biocatalytique, complètement indépendante de la première réaction biocatalytique, en combinant un second biocatalyseur immobilisé sur des particules magnétiques avec la seconde cuve réactionnelle contenant le mélange réactionnel biocatalytique transféré depuis la première cuve réactionnelle ; et
(d) la répétition des étapes (a) à (c) pour effectuer une série séquentielle de réactions biocatalytiques distinctes et indépendantes, et pour produire une série correspondante de composés de départ modifiés.

24. Procédé selon la revendication 23, dans lequel la réaction biocatalytique est effectuée avec un biocatalyseur immobilisé sur une particule, et le biocatalyseur est soustrait du mélange réactionnel biocatalytique par centrifugation ou par filtration.

25. Procédé selon la revendication 12, utilisant un dispositif automatique robotisé, le dispositif automatique robotisé se composant :
(a) d'une table XY comportant un bras de pipetage XYZ qui lui est lié, pour additionner des quantités volumétriques d'enzyme, de substrat, de cofacteur, de solutions de solvant et de solutions de réactifs à partir de récipients de réactifs placés sur la table XY, dans des cuves de réaction et de dosage placés sur la même table XY ;
(b) d'un bras de transfert XYZ de cuve de réaction, lié à la même table XY, utilisé pour le transfert des cuves de réaction et de dosages placées sur la table XY, vers différentes positions sur la table XY ;
(c) d'un bloc thermique d'incubation lié à la même table XY, pour loger les cuves de réaction et de dosage au cours des réactions d'incubation et pour contrôler la température des mélanges réactionnels ;
(d) d'un bloc de séparation magnétique lié à la même table XY, pour séparer le biocatalyseur immobilisé sur des particules magnétiques, du mélange biocatalytique, en appliquant un champ magnétique externe qui provoque l'attraction des particules magnétiques et leur concentration sur la source du champ magnétique, ce qui les sépare efficacement de la masse du mélange réactionnel biocatalytique ; et
(e) d'un microprocesseur programmable assurant l'interface avec le bras de pipetage XYZ et le bras XYZ de transfert de cuve de réaction, le bloc thermique et le bloc de séparation magnétique, de manière à contrôler et réguler avec précision tous les mouvements et opérations de ces unités fonctionnelles lors de la mise en oeuvre des réactions biocatalytiques destinées à produire des composés de départ modifiés, et des dosages destinés à déterminer les activités désirées.

26. Procédé selon la revendication 1, comprenant :
(a) le mélange d'un composé de départ médicamenteux avec un récepteur de haute affinité, à approximativement la moitié de la concentration molaire dudit composé de départ médicamenteux pour permettre à pratiquement la totalité dudit récepteur de haute affinité, de se lier au composé de départ médicamenteux et de laisser une quantité molaire égale du composé de départ médicamenteux, libre en solution et disponible pour la modification biocatalytique ;
(b) la production d'un composé médicamenteux modifié à partir de la réaction biocatalytique entre le composé médicamenteux de départ et le récepteur, le composé médicamenteux modifié ayant une plus forte affinité de liaison que le composé médicamenteux de départ ;
(c) le déplacement du composé médicamenteux de départ depuis le récepteur, avec le composé médicamenteux modifié ;
(d) la protection du composé médicamenteux modifié, lié, vis-à-vis des réactions biocatalytiques ultérieures ;
(e) le dissociation du complexe (composé médicamenteux modifié)-(récepteur) en ses parties composantes ;
(f) la détermination de la composition chimique du composé médicamenteux de départ, modifié ; et
(g) la répétition des étapes (a) à (e), et ainsi la mise en oeuvre d'une série de réactions biocatalytiques sur un composé médicamenteux de départ pour produire une banque de composés médicamenteux modifiés ayant des affinités de liaison plus fortes.

27. Procédé selon la revendication 26, dans lequel le récepteur de haute affinité est présent sur la surface, ou est contenu au sein, d'une cellule vivante, ou immobilisé sur un support naturel ou artificiel.

28. Procédé selon la revendication 4, dans lequel le contrôle de la banque de composés de départ modifiés est choisi au sein d'un groupe de dosages comprenant ceux qui sont énumérés dans le Tableau VI.

29. Procédé selon la revendication 12, dans lequel la réaction biocatalytique est effectuée avec un biocatalyseur immobilisé sur des particules magnétiques formant un biocatalyseur magnétique tandis que :
(a) la réaction biocatalytique est initiée en combinant le biocatalyseur immobilisé, avec ce qui suit, pour former le mélange réactionnel biocatalytique : substrat(s), cofacteur(s) et conditions solvant/ - tampon mises en oeuvre pour la réaction biocatalytique spécifique ;
(b) la réaction biocatalytique est achevée par le déplacement du biocatalyseur depuis le mélange réactionnel biocatalytique, ce qui est réalisé par l'application d'un champ magnétique externe qui provoque l'attraction des particules magnétiques avec le biocatalyseur immobilisé et leur concentration sur la source du champ magnétique, ce qui sépare efficacement le biocatalyseur magnétique de la masse du mélange réactionnel biocatalytique et qui permet le transfert du mélange réactionnel sans le biocatalyseur magnétique, vers une seconde cuve réactionnelle, en laissant le biocatalyseur magnétique dans la première cuve réactionnelle ;
(c) une seconde réaction biocatalytique, complètement indépendante de la première réaction biocatalytique, est initiée par introduction d'un second biocatalyseur immobilisé sur des particules magnétiques, dans une seconde cuve de réaction contenant le mélange réactionnel biocatalytique issu de la première réaction biocatalytique, sans le biocatalyseur magnétique utilisé dans la première réaction biocatalytique ;
(d) le cycle de réaction biocatalytique décrit ci-dessus est répété pour réaliser ainsi une série de réactions biocatalytiques distinctes et indépendantes, produisant une série correspondante de composés médicamenteux modifiés ;
(e) un bloc thermique de séparation lié à la même table XY que celle évoquée ci-dessus, pour séparer le biocatalyseur immobilisé sur des particules magnétiques provenant du premier mélange réactionnel, par l'application d'un champ magnétique externe qui provoque l'attraction des particules magnétiques et leur concentration sur la source du champ magnétique, ce qui les sépare efficacement de la masse du mélange réactionnel biocatalytique ; et
(f) un microprocesseur programmable assurant l'interface avec le bras de pipetage XYZ et le bras de transfert XYZ de transfert de cuve de réaction, le bloc thermique et le bloc de séparation magnétique, de manière à contrôler et réguler avec précision tous les mouvements et opérations de ces unités fonctionnelles lors de la mise en oeuvre des réactions biocatalytiques destinées à produire des composés de départ modifiés, et des dosages destinés à déterminer les activités désirées.

30. Procédé selon l'une quelconque des revendications 1 à 29, dans lequel les composés de départ sont solubles dans le mélange réactionnel.

31. Procédé selon l'une quelconque des revendications 1 à 29, dans lequel les composés de départ et les dérivés qui en résultent sont solubles et restent solubles en vue de l'utilisation dans les procédures de criblage.
